# EUROPEAN PATENT SPECIFICATION

(11) **EP 1 981 509 B1**
(45) Date of publication and mention of the grant of the patent: **17.03.2010**
(21) Application number: 07705350.2
(22) Date of filing: 23.01.2007
(51) Int. Cl.: A61K 31/519, C07D 487/04

(54) **Pyrazolo[1,5-a]pyrimidine derivatives for use in treatment of Alzheimer's disease and related conditions**
Pyrazolo[1,5-a]pyrimidine Derivate zur Verwendung in der Behandlung von Morbus Alzheimer und verwandten Leiden
Dérivés de pyrazolo[1,5-a]pyrimidine pour leur utilisation dans le traitement de la maladie d'Alzheimer et de conditions apparentées

(30) Priority: 27.01.2006 GB 0601638
(43) Date of publication of application: 22.10.2008
(73) Proprietor: Merck Sharp & Dohme Limited, Hoddesdon, Hertfordshire EN11 9BU (GB); Merck Sharp & Dohme Corp., Rahway, NJ 07065 (US)
(72) Inventor: CHURCHER, Ian, Hertfordshire EN11 9BU (GB); HUNT, Peter, Alan, Hertfordshire EN11 9BU (GB); STANTON, Matthew, G., Rahway, New Jersey 07065 (US)
(74) Representative: Buchan, Gavin MacNicol
(86) International application number: PCT/GB2007/050036
(87) International publication number: WO 2007/085873

(56) References cited:
- EP-A- 1 514 558
- US-A1- 2006 135 537
- FRALEY MARK E ET AL: "Optimization of a pyrazolo[1,5-a]pyrimidine class of KDR kinase inhibitors: improvements in physical properties enhance cellular activity and pharmacokinetics." BIOORGANIC & MEDICINAL CHEMISTRY LETTERS 16 DEC 2002, vol. 12, no. 24, 16 December 2002 (2002-12-16), pages 3537-3541, XP002427976 ISSN: 0960-894X cited in the application
- LAU LIT-FUI ET AL: "Tau protein phosphorylation as a therapeutic target in Alzheimer's disease." CURRENT TOPICS IN MEDICINAL CHEMISTRY APR 2002, vol. 2, no. 4, April 2002 (2002-04), pages 395-415, XP002427977 ISSN: 1568-0266
- KURET J ET AL: "Pathways of tau fibrillization" BIOCHIMICA ET BIOPHYSICA ACTA. MOLECULAR BASIS OF DISEASE, AMSTERDAM, NL, vol. 1739, no. 2-3, 3 January 2005 (2005-01-03), pages 167-178, XP004688067 ISSN: 0925-4439

## Description

This invention relates to compositions for use in the treatment or prevention of neurodegenerative diseases such as Alzheimer's disease. In particular, there is disclosed a particular class of pyrazolo[1,5-a]pyrimidine derivatives which selectively inhibit microtubule affinity regulating kinase (MARK).

Alzheimer's disease (AD) is the most common cause of dementia in the elderly and is characterised by a decline in cognitive function, that progresses slowly and results in symptoms such as memory loss and disorientation. Death occurs, on average, 9 years after diagnosis. The incidence of AD increases with age, so that while about 5% of people over the age of 70 are sufferers, this figure increases to 20% of those over 80 years old.

Existing treatments exclusively target the primary symptoms of AD. Diseased neurons may release insufficient or excessive amounts of particular neurotransmitters, and so current drugs are aimed at increasing neurotransmitter levels or at reducing the stimulation of nerve cells by neurotransmitters. Although these drugs provide some improvement in the symptoms of AD, they fail to address the underlying cause of the disease.

The classic clinical and neuropathological features of AD consist of senile or neuritic plaques and tangled bundles of fibers (neurofibrillary tangles) [Verdile, G., et al, Pharm. Res. 50:397-409 (2004)]. In addition, there is a severe loss of neurons in the hippocampus and the cerebral cortex. Neuritic plaques are extracellular lesions, consisting mainly of deposits of β-amyloid peptide (Aβ), surrounded by dystrophic (swollen, damaged and degenerating) neurites and glial cells activated by inflammatory processes. In contrast, neurofibrillary tangles (NFTs) are intracellular clusters composed of a hyperphosphorylated form of the protein tau, which are found extensively in the brain (e.g. mainly in cortex and hippocampus in AD). Tau is a soluble cytoplasmic protein which has a role in microtubule stabilisation. Excessive phosphorylation of this protein renders it insoluble and leads to its aggregation into paired helical filaments, which in turn form NFTs.

The amyloid cascade hypothesis proposes that abnormal accumulation of Aβ peptides, particularly Aβ42, initiates a cascade of events leading to the classical symptoms of AD and ultimately, to the death of the patient. There is strong evidence [e.g. Rapoport, M., et al (2002) Proc. Natl. Acad. Sci USA 99:6364-6369] that dysregulation of tau function is a key step in the cascade of Alzheimer's disease pathology leading ultimately to neuronal death. Furthermore, tau mutations and NFTs are found in other dementias in which Aβ pathology is absent, such as frontotemporal dementia, Pick's disease and parkinsonism linked to chromosome 17 (FTDP-17) [Mizutani, T. (1999) Rinsho Shikeigaku 39: 1262-1263]. Also, in AD the frequency of NFTs correlates to the degree of dementia better than that of senile plaques [Arriagada, P.V., et al (1992) Neurology 42:631-639], while significant numbers of amyloid plaques are often found in the brains of non-demented elderly people, suggesting that amyloid pathology on its own is not sufficient to cause dementia. For these reasons, normalisation of tau function (in particular prevention of hyperphosphorylation) is seen as a desirable therapeutic goal for the treatment of AD and other dementing conditions.

Tau is a 352-441 amino acid protein encoded by the Mapt (*Microtubule-associated protein tau*) gene which is widely expressed in the central nervous system (CNS) with localisation primarily in axons [Binder et al J. Cell Biol. 1985, 101(4), 1371-1378]. The major function of tau is regulation of the stability of microtubules (MTs), intracellular structural components comprised of tubulin dimers which are integral in regulating many essential cellular processes such as axonal transport and elongation as well as generation of cell polarity and shape. Tau binding to tubulin is a key factor in determining the rates of polymerisation/depolymerisation (termed dynamic instability) of MTs, and tau is therefore key to the regulation of many essential cellular processes [see, for example, Butner, K.A., Kirschner, M.W. (1991) J.Cell. Biol. 115: 717-730].

Tau is a basic protein with numerous serine and threonine residues, many of which are susceptible to phosphorylation. While normal tau has two to three phosphorylated amino acid residues, hyperphosphorylated tau found in AD and other tauopathies typically has eight or nine phosphorylated residues. A variety of kinases promote phosphorylation of these sites, including proline-directed kinases such as glycogen synthase kinase 3β (GSK3β) and cyclin dependent kinase 5 (cdk5); and non-proline-directed kinases such as protein kinase A (PKA) and calmodulin (CaM) kinase II, which phosphorylate tau at Lys-(Ile/Cys)-Gly-Ser sequences, also known as KXGS motifs. One KXGS motif is found in each of the MT binding repeats. Phosphorylation at these sites is important for the regulation of tau-MT binding and while the degree of phosphorylation is normally low, it has been shown to be increased in brain tissue from AD patients. Phosphorylation of one particular residue within the KXGS motifs, Ser-262 has been shown to be elevated in tau protein extracted from the NFTs in AD [Hasegawa, M. et al (1992) J. Biol. Chem 267:17047-17054] and phosphorylation at this site also appears to dramatically reduce MT binding [Biernat, J. et al. (1993) Neuron 11: 153-163].

Nishimura et al. [Cell 116: 671-682 (2004)] demonstrated that overexpression of the kinase PAR-1 in *Drosophila* led to enhanced tau-mediated toxicity and an increase in the phosphorylation of tau on Ser-262, Ser-356, and other amino acid residues, including sites phosphorylated by GSK3β and Cdk5. Their findings suggest that PAR-1 kinase acts as a master kinase during the process of tau hyperphosphorylation, with the phosphorylation of the Ser-262 and Ser-356 sites being a prerequisite for the subsequent phosphorylation at downstream sites by other kinases.

The mammalian ortholog of PAR-1 is microtubule affinity-regulating kinase (MARK). There are four MARK isoforms and these form part of the AMP-dependent protein kinase (AMPK) family. Like PAR-1, MARK is thought to phosphorylate tau, perhaps in response to an external insult, such as the disruption of Ca²⁺ homeostasis caused by Aβ, priming it for further phosphorylation events. It is not clear whether the phosphorylation of tau by MARK leads directly to its detachment from MTs or the subsequent phosphorylation events cause detachment. The resulting unbound, hyperphosphorylated tau is delocalised to the somatodendritic compartment and is then cleaved by caspases to form fragments prone to aggregation [Drewes, G. (2004). Trends Biochem. Sci 29:548-555; Gamblin, T.C., et al, (2003) Proc. Natl. Acad. Sci. U.S.A. 100:10032-10037]. These aggregates can grow into filaments, which are potentially toxic, eventually forming the NFTs found in AD.

For these reasons, it is proposed that MARK inhibitors will enable the prevention or amelioration of neurodegeneration in AD and other tauopathies.

In WO 98/54093, WO 00/53605, WO 2004/052286, WO 2004/052315 and in Fraley et al, Biorg. Med. Chem. Lett., 12(2002) 3537-41, various 3,6-disubstituted pyrazolo[1,5-a]pyrimidines derivatives are disclosed as inhibitors of tyrosine kinases (e.g. KDR kinase), implicated in angiogenesis and other cell proliferative processes, but there is no disclosure of utility as MARK inhibitors or in the treatment or prevention of tauopathies.

According to the invention, there is provided the use, for the manufacture of a medicament for treatment or prevention of a neurodegenerative disease associated with hyperphosphorylation of tau, of a compound according to formula I: 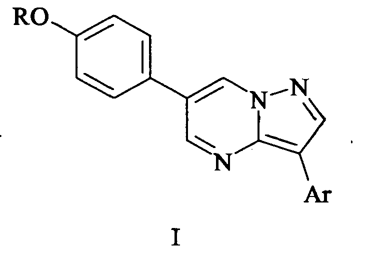 or a pharmaceutically acceptable salt or hydrate thereof; wherein:
R represents C₁₋₄alkyl which is optionally substituted with halogen, CN, CF₃, OR¹, NR¹R², NHPh or NHCOC₁₋₄alkyl; or R may complete a fused tetrahydrofuran ring;
Ar represents phenyl or optionally benzofused 5- or 6-membered heteroaryl, any of which optionally bears up to 3 independently-selected R³ substituents;
R¹ and R² independently represent H or C₁₋₄alkyl, or R¹ and R² bonded to the same nitrogen atom may complete a heterocyclic ring of up to 6 members which optionally comprises one additional heteroatom selected from N, O and S and which optionally bears up to 2 substituents selected from C₁₋₄alkyl, CN, CF₃, halogen and oxo;
R³ represents halogen, CN, R⁵, SR⁵, X-OR⁴, X-N(R⁴)₂, CH(CF₃)-N(R⁴)₂, COR⁴, CONHOH, phenyl, 5- or 6-membered heteroaryl or C-heterocyclyl, said phenyl, 5- or 6-membered heteroaryl or C-heterocyclyl optionally bearing up to 2 substituents selected from C₁₋₄alkyl, CF₃ and halogen; or when Ar represents phenyl two R³ groups attached to adjacent ring atoms on Ar may complete a fused 5- or 6-membered carbocyclic or heterocyclic ring which optionally bears up to 3 substituents selected from oxo, imino, and R⁵_{;}
R⁴ represents H, CF₃, CH(CF₃)-Ar¹, or alkyl, alkenyl, cycloalkyl or cycloalkylalkyl of up to 6 carbon atoms which is optionally substituted with halogen, CN, CF₃, OR¹ or NR¹R²; or two R⁴ groups bonded to the same nitrogen atom may complete a heterocyclic ring of up to 6 members which optionally comprises one additional heteroatom selected from N, O and S and which optionally bears up to 2 substituents selected from C₁₋₄alkyl, CF₃, halogen and oxo;
R⁵ represents R⁴ that is not H;
Ar¹ represents an aromatic mono- or bicyclic ring system of up to 10 ring atoms of which 0-3 are selected from N, O and S and the rest are carbon, said ring system bearing 0-3 substituents selected from halogen, CF₃ and C₁₋₄alkyl;
X represents a bond, CH₂ or CO; and
"C-heterocyclyl" refers to nonaromatic heterocyclic rings of 5 or 6 ring atoms, up to 2 of which are selected from N, O and S, said ring being attached via a ring carbon atom.

In a particular embodiment,
R represents C₁₋₄alkyl which is optionally substituted with halogen, CN, CF₃, OR¹ or NR¹R²;
R³ represents halogen, CN, R⁵, SR⁵, X-OR⁴, X-N(R⁴)₂, COR⁴, CONHOH, phenyl, 5- or 6-membered heteroaryl or C-heterocyclyl, said phenyl, 5- or 6-membered heteroaryl or C-heterocyclyl optionally bearing up to 2 substituents selected from C₁₋₄alkyl, CF₃ and halogen; or when Ar represents phenyl two R³ groups attached to adjacent ring atoms on Ar may complete a fused 5- or 6-membered carbocyclic or heterocyclic ring which optionally bears up to 3 substituents selected from oxo, imino, and R⁵;
and R⁴ represents H, CF₃ or alkyl, alkenyl, cycloalkyl or cycloalkylalkyl of up to 6 carbon atoms which is optionally substituted with halogen, CN, CF₃, OR¹ or NR¹R²; or two R⁴ groups bonded to the same nitrogen atom may complete a heterocyclic ring of up to 6 members which optionally comprises one additional heteroatom selected from N, O and S and which optionally bears up to 2 substituents selected from C₁₋₄alkyl, CF₃, halogen and oxo.

The invention further provides compounds of formula I for use in treatment or prevention of a neurodegenerative disease associated with hyperphosphorylation of tau in a human patient.

"Neurodegenerative diseases associated with hyperphosphorylation of tau" refers to AD, frontotemporal dementia, Pick's disease and parkinsonism linked to chromosome 17 (FTDP-17).

As used herein, the expression "C₁₋ₓalkyl" where x is an integer greater than 1 refers to straight-chained and branched alkyl groups wherein the number of constituent carbon atoms is in the range 1 to x. Particular alkyl groups are methyl, ethyl, n-propyl, isopropyl and t-butyl. Derived expressions such as "C₂₋₆alkenyl", "hydroxyC₁₋₆alkyl", "heteroarylC₁₋₆alkyl", "C₂₋₆alkynyl" and "C₁₋₆alkoxy" are to be construed in an analogous manner. Most suitably, the number of carbon atoms in such groups is not more than 6.

The term "halogen" as used herein includes fluorine, chlorine, bromine and iodine.

The expression "C₃₋₆cycloalkyl" as used herein refers to nonaromatic monocyclic hydrocarbon ring systems comprising from 3 to 6 ring atoms. Examples include cyclopropyl, cyclobutyl, cyclopentyl and cyclohexyl.

For use in medicine, the compounds of formula I may be in the form of pharmaceutically acceptable salts. Other salts may, however, be useful in the preparation of the compounds of formula I or of their pharmaceutically acceptable salts. Suitable pharmaceutically acceptable salts of the compounds of this invention include acid addition salts which may, for example, be formed by mixing a solution of the compound according to the invention with a solution of a pharmaceutically acceptable acid such as hydrochloric acid, sulphuric acid, methanesulphonic acid, benzenesulphonic acid, fumaric acid, maleic acid, succinic acid, acetic acid, trifluoroacetic acid, benzoic acid, oxalic acid, citric acid, tartaric acid, carbonic acid or phosphoric acid. Alternatively, where the compound of the invention carries an acidic moiety, a pharmaceutically acceptable salt may be formed by neutralisation of said acidic moiety with a suitable base. Examples of pharmaceutically acceptable salts thus formed include alkali metal salts such as sodium or potassium salts; ammonium salts; alkaline earth metal salts such as calcium or magnesium salts; and salts formed with suitable organic bases, such as amine salts (including pyridinium salts) and quaternary ammonium salts.

When the compounds useful in the invention have one or more asymmetric centres, they may accordingly exist as enantiomers. Where the compounds according to the invention possess two or more asymmetric centres, they may additionally exist as diastereoisomers. It is to be understood that all such isomers and mixtures thereof in any proportion are encompassed within the scope of the present invention.

When a compound useful in the invention is capable of existing in tautomeric keto and enol forms, both of said forms are considered to be within the scope of the invention.

A nitrogen atom forming part of a heteroaryl ring may be in the form of the N-oxide. A sulphur atom forming part of a nonaromatic heterocycle may be in the form of the S-oxide or S,S-dioxide.

A heteroaryl group may be attached to the remainder of the molecule via a ring carbon or a ring nitrogen, provided that this is consistent with preservation of aromaticity.

In formula I, R may complete a fused tetrahydrofuran ring, but preferably R represents C₁₋₄ alkyl which is optionally substituted with halogen, CN, CF₃, OR¹, NR¹R², NHPh or NHCOC₁₋₄alkyl, where R¹ and R² are as defined previously. In one embodiment, R represents unsubstituted C₁₋₄alkyl, in particular methyl. When R represents substituted C₁₋₄alkyl, a preferred substituent is NR¹R², and in a particular embodiment R represents CH₂CH₂NR¹R² or CH₂CH₂CH₂NR¹R². R¹ and R² independently represent H or C₁₋₄alkyl such as methyl, or together complete a heterocyclic ring of up to 6 members. Suitable rings completed by R¹ and R² include pyrrolidine, piperidine, piperazine and morpholine. Specific examples of groups represented by R include methyl, 2-(pyrrolidin-1-yl)ethyl, 2-(piperidin-1-yl)ethyl, 2-(morpholin-4-yl)ethyl, 2-(4-cyanopipendin-1-yl)ethyl, 3-(dimethylamino)propyl, 2-(dimethylamino)ethyl, 2-(acetylamino)ethyl, 2-(methylamino)ethyl, 2-(phenylamino)ethyl, 2-(4-methylpiperazin-1-yl)ethyl, 2-(3,3-difluoropipetidine-1-yl)ethyl, 2-(3-fluoropyrrolidin-1-yl)ethyl, 2-(4,4-difluoropiperidin-1-yl)ethyl, 2-methoxyethyl and 3-methoxypropyl.

Ar represents phenyl or optionally benzofused 5- or 6-membered heteroaryl, any of which may bear up to 3 independently selected R³ substituents as defined previously. Preferably, Ar is monosubstituted or disubstituted. Examples of 5-membered heteroaryl rings represented by Ar include thiophene and benzofuran, and examples of 6-membered heteroaryl rings represented by Ar include pyridine. In a particular embodiment Ar represents optionally substituted 3-thienyl.

When Ar represents phenyl and two R³ groups are present on adjacent ring carbons, said R³ groups may combine to form a fused 5- or 6-membered carbocylic or heterocyclic ring which optionally bears up to 3 substituents selected from oxo, imino, and R⁵ where R⁵ is as defined previously. For example, the R³ groups may complete a pyrrolidine ring so that Ar represents an isoindolinyl group, in particular a 3-iminoisoindolin-1-one group or an isoindolin-1,3-dione group, optionally substituted on the 2-position, wherein said 2-substituent is an optionally-substituted C₁₋₄alkyl group such as methyl, ethyl, trifluoroethyl, hydroxyethyl or dimethylaminoethyl.

Preferred substituents represented by R³ include halogen (especially Cl or F), CN, OR⁴, CH₂OR⁴, CH(CF₃)-N(R⁴)₂, CO₂R⁴, COR⁴, CON(R⁴)₂, CH₂N(R⁴)₂, pyridyl and 5-membered heteroaryl (such as furyl, thienyl and pyrazolyl), where R⁴ is as defined previously. Suitable identities for R⁴ include H, C₁₋₄alkyl (optionally substituted with CF₃, OR¹ or NR¹R²), allyl, cyclopropyl, cyclopropylmethyl and cyclobutyl. Another favoured identity for R⁴ is CH(CF₃)-Ar¹ where Ar¹ is as defined previously. Suitable identities for Ar¹ include furyl, pyridyl, imidazolyl, quinolyl and benzothiophenyl. In a particular embodiment Ar bears a substituent CONHR⁴ where R⁴ is H or C₁₋₄alkyl which is optionally substituted with CF₃, OR¹ or NR¹R², or where R⁴ is CH(CF₃)-Ar¹.

A subset of the compounds suitable for use in the invention consists of those in accordance with formula II: 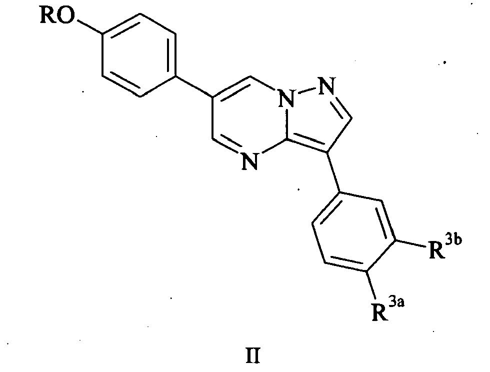 and pharmaceutically acceptable salts and hydrates thereof; wherein R^{3a} and R^{3b} independently represent H or R³, and R and R³ have the same definitions and preferred identities as described previously. Specific examples of compounds in accordance with formula II are listed in Table 1:

**Table1**

| **R** | **R^{3a}** | **R^{3b}** |
|---|---|---|
| 2-(piperidin-1-yl)ethyl | H | H |
| 2-(piperidin-1-yl)ethyl | F | H |
| 2-(piperidin-1-yl)ethyl | H | NHAc |
| 2-(piperidin-1-yl)ethyl | H | morpholin-4-ylmethyl |
| 2-(piperidin-1-yl)ethyl | H | OEt |
| 2-(piperidin-1-yl)ethyl | Ac | H |
| 2-(piperidin-1-yl)ethyl | CN | H |
| 2-(piperidin-1-yl)ethyl | F | F |
| Me | CO₂H | H |
| 2-(piperidin-1-yl)ethyl | Cl | H |
| 2-(piperidin-1-yl)ethyl | OMe | H |
| 2-(piperidin-1-yl)ethyl | CO₂H | H |
| 2-(piperidin-1-yl)ethyl | H | OMe |
| 2-(piperidin-1-yl)ethyl | SMe | H |
| 2-(piperidin-1-yl)ethyl | CH₂OH | H |
| 2-(piperidin-1-yl)ethyl | OH | H |
| 2-(piperidin-1-yl)ethyl | CONHMe | H |
| 2-(piperidin-1-yl)ethyl | CONHEt | H |
| 2-(piperidin-1-yl)ethyl | CONH₂ | H |
| 2-(piperidin-1-yl)ethyl | CONHCH₂CH₂OH | H |
| 2-(piperidin-1-yl)ethyl | CONHCH₂CH₂NH₂ | H |
| 2-(piperidin-1-yl)ethyl | CONHOH | H |
| 2-(piperidin-1-yl)ethyl | CN | OH |
| 2-(piperidin-1-yl)ethyl | CN | OAc |
| 2-(piperidin-1-yl)ethyl | CN | 3-pyridyl |
| 2-(piperidin-1-yl)ethyl | CN | CO₂Me |
| 2-(piperidin-1-yl)ethyl | CN | CON(Me)₂ |
| 2-(piperidin-1-yl)ethyl | CN | 2-thienyl |
| 2-(piperidin-1-yl)ethyl | CN | 3-pyrazolyl |
| 2-(piperidin-1-yl)ethyl | CN | 3-furyl |
| 2-(piperidin-1-yl)ethyl | CN | CONH₂ |
| 2-(piperidin-1-yl)ethyl | CN | CON(Me)CH₂CH₂N(Me)₂ |
| 2-(piperidin-1-yl)ethyl | CN | CO-(1-pyrrolidinyl) |
| 2-(piperidin-1-yl)ethyl | CN | CO-(1-piperidinyl) |
| 2-(piperidin-1-yl)ethyl | CN | O-allyl |
| 2-(piperidin-1-yl)ethyl | CO₂H | CO₂H |
| 2-(piperidin-1-yl)ethyl | CN | OMe |
| 2-(piperidin-1-yl)ethyl | CN | OCH₂CH₂N(Me)₂ |
| 2-(piperidin-1-yl)ethyl | CN | OCH₂CH₂(1-pyrrolidinyl) |
| 2-(piperidin-1-yl)ethyl | CN | OCH₂CH₂NH-n-propyl |
| 2-(piperidin-1-yl)ethyl | CONHCH₂CF₃ | H |
| 2-(piperidin-1-yl)ethyl | H | CONHCH(CP₃)-ⁱPr |
| 2-(piperidin-1-yl)ethyl | H | CONHCH₂CF₃ |

A subset of the compounds of formula II consists of those in accordance with formula IIA: 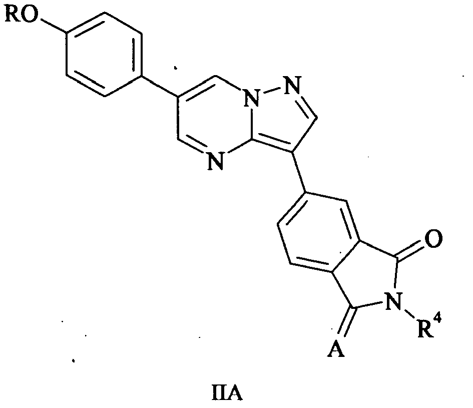 wherein A represents O or NH, and R and R⁴ have the same definitions and preferred identities as before. Specific examples of compounds in accordance with formula IIA include those in which R represents 2-(piperidin-1-yl)ethyl and A and R⁴ areas indicated in Table 2:

**Table 2**

| **A** | **R⁴** |
|---|---|
| NH | Me |
| NH | Et |
| NH | CH₂CH₂OH |
| NH | CH₂CF₃ |
| NH | CH₂CH₂N(Me)₂ |
| NH | CH₂CH₂NH₂ |
| O | H |

Another subset of the compounds suitable for use in the invention consists of those in accordance with formula III: 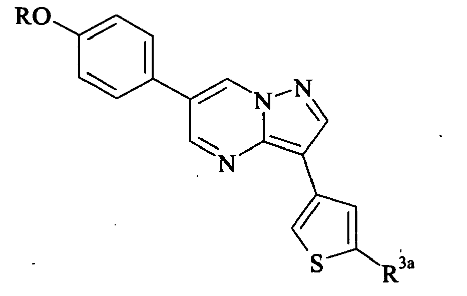 and pharmaceutically acceptable salts and hydrates thereof; wherein R^{3a} represents H or R³, and R and R³ have the same definitions and preferred identities as described previously. Specific examples of compounds in accordance with formula III include those in which R and R^{3a} are as listed in Table 3:

**Table 3**

| **R** | **R^{3a}** |
|---|---|
| Me | CONHMe |
| Me | CONHEt |
| Me | CONH cyclobutyl |
| Me | CONH cyclopropyl |
| Me | CONH-n-propyl |
| 2-(morpholin-4-yl)ethyl | H |
| 2-(piperidin-1-yl)ethyl | H |
| 2-(piperidin-1-yl)ethyl | CO₂Me |
| 2-(piperidin-1-yl)ethyl | CONHMe |
| 2-(piperidin-1-yl)ethyl | CONHCH₂CH₂OH |
| 2-(piperidin-1-yl)ethyl | CONH₂ |
| 2-(piperidin-1-yl)ethyl | CONHEt |
| 2-(piperidin-1-yl)ethyl | CONH-isobutyl |
| 2-(piperidin-1-yl)ethyl | CON(Me)₂ |
| 2-(piperidin-1-yl)ethyl | CONHCH₂CH₂NH₂ |
| 2-(piperidin-1-yl)ethyl | CO-(1-pyrrolidinyl) |
| 2-(piperidin-1-yl)ethyl | CO-(1-piperidinyl) |
| 2-(piperidin-1-yl)ethyl | CONHCH₂CF₃ |
| 2-(piperidin-1-yl)ethyl | CONHOH |
| 2-(piperidin-1-yl)ethyl | CONHCH₂CH₂N(Me)₂ |
| 2-(piperidin-1-yl)ethyl | CON(Me)CH₂CH₂N(Me)₂ |
| 2-(piperidin-1-yl)ethyl | CH₂NH-isobutyl |
| 2-(piperidin-1-yl)ethyl | CHO |
| 2-(piperidin-1-yl)ethyl | CH₂NHCH₂CH₂OH |
| 2-(piperidin-1-yl)ethyl | CH₂NHCH₂CH₂N(Me)₂ |
| 2-(piperidin-1-yl)ethyl | CH₂N(Me)CH₂CH₂N(Me)₂ |
| 2-(piperidin-1-yl)ethyl | 4-isopropyl-4,5-dihydro-1,3-oxazol-2-yl |
| 2-(dimethylamino)ethyl | CH₂CF₃ |
| 2-(pyrrolidin-1-yl)ethyl | CH₂CF₃ |
| 2-(piperidin-1-yl)ethyl | CH₂NHCH₂CH₂NH₂ |
| 2-(piperidin-1-yl)ethyl | CH₂NHCH₂CF₃ |
| 2-(piperidin-1-yl)ethyl | CH₂OH |
| 2-(piperidin-1-yl)ethyl | 5-methyl-4,5-dihydro-1*H*-imidazol-2-yl |

Further compounds in accordance with formula III include those in which R and R^{3a} are as shown in Table 3A:

| **R** | **R^{3a}** |
|---|---|
| 2-(morpholin-4-yl)ethyl | CONHCH₂CF₃ |
| 2-(4-Me-piperazin-1-yl)ethyl | CONHCH₂CF₃ |
| 2-(piperidin-1-yl)ethyl | CONHCH(CF₃)-(2-furyl) |
| 2-(piperidin-1-yl)ethyl | CONHCH(CF₃)-(2-pyridyl) |
| 2-(piperidin-1-yl)ethyl | CH₂NHCH(CF₃)-(2-furyl) |
| 2-(piperidin-1-yl)ethyl | CH₂NHCH(CF₃)-(2-pyridyl) |
| 2-(piperidin-1-yl)ethyl | CH(CF₃)-NH2 |
| 2-(piperidin-1-yl)ethyl | CONHCH(CF₃)-Me |
| 2-(piperidin-1-yl)ethyl | CH(CF₃)-NH-isopropyl |
| 2-(piperidin-1-yl)ethyl | CH(CF₃)-NHCH₂cyclopropyl |
| 2-(piperidin-1-yl)ethyl | CONHC(Me)₂CF₃ |
| 2-(3,3-di-F-piperidin-1-yl)ethyl | CONHCH₂CF₃ |
| 2-(3-F-pyrrolidin-1-yl) | CONHCH₂CF₃ |
| 2-(piperidin-1-yl)ethyl | CONHCH(CF₃)-(3-pyridyl) |
| 2-(3-F-piperidin-1-yl)ethyl | CONHCH₂CF₃ |
| 2-(piperidin-1-yl)ethyl | CONHCH(CF₃)-isopropyl |
| 2-(3,3-di-F-pyrtolidin-1yl)ethyl | CONHCH₂CF₃ |
| 2-(4,4-di-F-piperidin-lyl)ethyl | CONHCH₂CF₃ |
| 2-(piperidin-1-yl)ethyl | CONHCH(CF₃)-(quinolin-5-yl) |
| 2-(piperidin-1-yl)ethyl | CONHCH(CF₃)-(quinolin-8-yl) |
| 2-(piperidin-1-yl)ethyl | CH₂NHCH(CF₃)-isopropyl |
| 2-(piperidin-1-yl)ethyl | CONHCH(CF₃)-(1-Me-imidazol-2-yl) |
| 2-(piperidin-1-yl)ethyl | CONHCH(CF₃)-(4-pyridyl) |
| 2-(piperidin-1-yl)ethyl | CONHCH(CF₃)-(benzthiophen-2-yl) |
| 2-AcNH-ethyl | CONHCH(CF₃)-isopropyl |
| Fused tetrahydrofuran | CONHCH₂CF₃ |
| 2-methoacyethyl | CONHCH₂CF₃ |
| 3-methoxypropyl | CONHCH₂CF₃ |

Compounds of formula III in which R represents substituted C₁₋₄alkyl and R^{3a} is other than H, and the pharmaceutically acceptable salts and hydrates thereof, are believed to be novel, and therefore constitute a further aspect of the invention. The invention further extends to pharmaceutical compositions comprising a compound of formula III in which R represents substituted C₁₋₄alkyl and R^{3a} is other than H, or a pharmaceutically acceptable salt or hydrate thereof, and a pharmaceutically acceptable carrier.

Preferably R represents substituted C₁₋₄alkyl in which the substituent is NR¹R², and in a particular embodiment R represents CH₂CH₂NR¹R² or CH₂CH₂CH₂NR¹R², where R¹ and R² have the same definitions and preferred identities as before, for example 2-(piperidin-1-yl)ethyl.

In a particular embodiment, R^{3a} represents CON(R⁴)₂ where R⁴ has the same definition and preferred identities as before. Very suitably, R^{3a} represents CONHR⁴ where R⁴ is H or C₁₋₄alkyl which is optionally substituted with CF₃, OR¹ or NR¹R², for example CONHCH₂CF₃.

In another embodiment, R^{3a} represents CH(CF₃)NHR⁴ and R⁴ represents H or C₁₋₄alkyl which is optionally substituted with CF₃, OR¹ or NR¹R².

In a further embodiment, R^{3a} represents CH₂NHR⁴ or CONHR⁴ and R⁴ represents CH(CF₃)-Ar¹ where Ar¹ is as defined previously. For example, Ar¹ may represent 5- or 6-membered heteroaryl, such as imidazolyl, quinolyl, benzothiophenyl, furyl or pyridyl, in particular 2-furyl or 2-pyridyl. Further specific examples of compounds of formula I include those in which R and Ar are as shown in Table 4:

**Table 4**

| **R** | **Ar** |
|---|---|
| 2-(pyrrolidin-1-yl)ethyl | 4-pyridyl |
| 2-(piperidin-1-yl)ethyl | 2-thienyl |
| 2-(piperidin-1-yl)ethyl | 4-pyridyl |
| 3-(dimethylamino)propyl | 4-pyridyl |
| 2-(4-cyanopiperidin-1-yl)ethyl | 3-pyridyl |
| 2-(piperidin-1yl)ethyl | benzofuran-2-yl |
| 2-(piperidin-1-yl)ethyl | 6-fluoro-3-pyridyl |
| 2-(piperidin-1-yl)ethyl | 6-methoxy-3-pyridyl |
| 2-(piperidin-1-yl)ethyl | 6-amino-3-pyridyl |

Compounds in accordance with formula I may be prepared by the methods disclosed in the aforementioned WO 98/54093, WO 00/53605, WO 2004/052286, WO 2004/052315 and Fraley et al, Biorg. Med. Chem. Lett., 12(2002) 3537-41, or by straightforward adaptations thereof. Typically, a compound of formula I is obtained by Suzuki coupling of Ar-B(OR')₂ with a compound of formula (1): 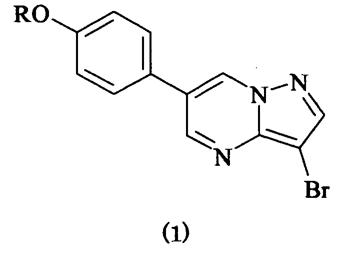 where R' represents H or C₁₋₄alkyl, or the two OR' groups complete a cyclic boronate ester, and R and Ar have the same meanings as before. The reaction takes place under standard Suzuki conditions, e.g. in aqueous dioxan at 100°C in the presence of Pd(PPh₃)₄ and a base such as sodium carbonate. The relevant boronic acids and esters are either available commercially or are accessible by standard methods, e.g. treatment of Ar-Br with dipinacoldiborane in the presence of PdCl₂(dppf) and potassium acetate in dioxan at about 85°C.

Compounds (1) are obtainable by alkylation of the corresponding phenol (2) with R-L: 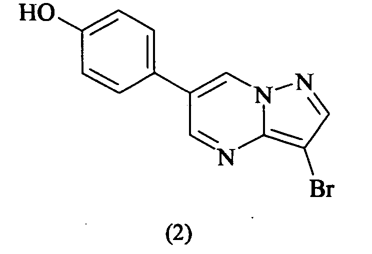 where L is a leaving group (e.g. Cl, Br, mesylate or tosylate) and R has the same meaning as before. In a typical procedure, the compound of formula (2) is treated with R-Cl in DMF in the presence of caesium carbonate and sodium iodide at about 60°C. The synthesis of compound (2) is described in the Examples section appended hereto.

It will be readily apparent that individual compounds in accordance with formula I may be converted into other compounds in accordance with formula I by means of standard techniques of synthetic chemistry familiar to those skilled in the art. For example, compounds in which Ar bears a CO₂C₁₋₄alkyl substituent may be hydrolysed to the corresponding acids then coupled with (R⁴)₂NH to provide compounds I which Ar bears a substituent CON(R⁴)₂ where R⁴ is has the same meaning as before. The coupling may be carried out using standard coupling techniques, e.g. using agents such benzotriazol-1-yloxytris(dimethylamino)phosphonium hexafluorophosphate (BOP).

Where they are not themselves commercially available, the starting materials and reagents described above may be obtained from commercially available precursors by means of well known synthetic procedures and/or the methods disclosed in the Examples section herein.

Where the above-described processes for the preparation of the compounds of use in the invention give rise to mixtures of stereoisomers, these isomers may be separated by conventional techniques such as preparative chromatography. The compounds may be prepared in racemic form, or individual enantiomers may be prepared either by enantiospecific synthesis or by resolution. The compounds may, for example, be resolved into their component enantiomers by standard techniques such as preparative HPLC, or the formation of diastereomeric pairs by salt formation with an optically active acid, such as di-*p*-toluoyl-D-tartaric acid and/or di-*p*-toluoyl-L-tartaric acid, followed by fractional crystallization and regeneration of the free base. The compounds may also be resolved by formation of diastereomeric esters or amides, followed by chromatographic separation and removal of the chiral auxiliary.

During any of the above synthetic sequences it may be necessary and/or desirable to protect sensitive or reactive groups on any of the molecules concerned. This may be achieved by means of conventional protecting groups, such as those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.

The compounds of formula I are suitably administered to patients in the form a pharmaceutical composition comprising the active ingredient (i.e. the compound of formula I or pharmaceutically acceptable salt or hydrate thereof) and a pharmaceutically acceptable carrier.

Preferably these compositions are in unit dosage forms such as tablets, pills, capsules, powders, granules, sterile parenteral solutions or suspensions, metered aerosol or liquid sprays, drops, ampoules, transdermal patches, auto-injector devices or suppositories; for oral, parenteral, intranasal, sublingual or rectal administration, or for administration by inhalation or insufflation. The principal active ingredient typically is mixed with a pharmaceutical carrier, e.g. conventional tableting ingredients such as corn starch, lactose, sucrose, sorbitol, talc, stearic acid, magnesium stearate and dicalcium phosphate, or gums, dispersing agents, suspending agents or surfactants such as sorbitan monooleate and polyethylene glycol, and other pharmaceutical diluents, e.g. water, to form a homogeneous preformulation composition containing a compound of the present invention, or a pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This preformulation composition is then subdivided into unit dosage forms of the type described above containing from 0.1 to about 500 mg of the active ingredient of the present invention. Typical unit dosage forms contain from 1 to 100 mg, for example 1, 2, 5, 10, 25, 50 or 100 mg, of the active ingredient. Tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compositions useful in the present invention may be incorporated for administration orally or by injection include aqueous solutions, liquid- or gel-filled capsules, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, poly(ethylene glycol), poly(vinylpyrrolidone) or gelatin.

In one embodiment of the invention, the compound of formula I is administered to a patient suffering from AD, FTDP-17, Pick's disease or frontotemporal dementia, preferably AD.

In an alternative embodiment of the invention, the compound of formula I is administered to a patient suffering from mild cognitive impairment or age-related cognitive decline. A favourable outcome of such treatment is prevention or delay of the onset of AD. Age-related cognitive decline and mild cognitive impairment (MCI) are conditions in which a memory deficit is present, but other diagnostic criteria for dementia are absent (Santacruz and Swagerty, American Family Physician, 63 (2001), 703-13). (See also "The ICD-10 Classification of Mental and Behavioural Disorders", Geneva: World Health Organisation, 1992, 64-5). As used herein, "age-related cognitive decline" implies a decline of at least six months' duration in at least one of: memory and learning, attention and concentration; thinking; language; and visuospatial functioning and a score of more than one standard deviation below the norm on standardized neuropsychologic testing such as the MMSE. In particular, there may be a progressive decline in memory. In the more severe condition MCI, the degree of memory impairment is outside the range considered normal for the age of the patient but AD is not present. The differential diagnosis of MCI and mild AD is described by Petersen et al., Arch. Neurol., 56 (1999), 303-8. Further information on the differential diagnosis of MCI is provided by Knopman et al, Mayo Clinic Proceedings, 78 (2003), 1290-1308. In a study of elderly subjects, Tuokko et al (Arch, Neurol., 60 (2003) 577-82) found that those exhibiting MCI at the outset had a three-fold increased risk of developing dementia within 5 years.

Grundman et al (J. Mol. Neurosci., 19 (2002), 23-28) report that lower baseline hippocampal volume in MCI patients is a prognostic indicator for subsequent AD. Similarly, Andreasen et al (Acta Neurol. Scand, 107 (2003) 47-51) report that high CSF levels of total tau, high CSF levels of phospho-tau and lowered CSF levels of Aβ42 are all associated with increased risk of progression from MCI to AD.

Within this embodiment, the compound of formula I is advantageously administered to patients who suffer impaired memory function but do not exhibit symptoms of dementia. Such impairment of memory function typically is not attributable to systemic or cerebral disease, such as stroke or metabolic disorders caused by pituitary dysfunction. Such patients may be in particular people aged 55 or over, especially people aged 60 or over, and preferably people aged 65 or over. Such patients may have normal patterns and levels of growth hormone secretion for their age. However, such patients may possess one or more additional risk factors for developing Alzheimer's disease. Such factors include a family history of the disease; a genetic predisposition to the disease; elevated serum cholesterol; and adult-onset diabetes mellitus.

In a particular embodiment of the invention, the compound of formula I is administered to a patient suffering from age-related cognitive decline or MCI who additionally possesses one or more risk factors for developing AD selected from: a family history of the disease; a genetic predisposition to the disease; elevated serum cholesterol; adult-onset diabetes mellitus; elevated baseline hippocampal volume; elevated CSF levels of total tau; elevated CSF levels of phospho-tau; and lowered CSF levels of Aβ(1-42).

A genetic predisposition (especially towards early onset AD) can arise from point mutations in one or more of a number of genes, including the APP, presenilin-1 and presenilin-2 genes. Also, subjects who are homozygous for the ε4 isoform of the apolipoprotein E gene are at greater risk of developing AD.

The patient's degree of cognitive decline or impairment is advantageously assessed at regular intervals before, during and/or after a course of treatment in accordance with the invention, so that changes therein may be detected, e.g. the slowing or halting of cognitive decline. A variety of neuropsychological tests are known in the art for this purpose, such as the Mini-Mental State Examination (MMSE) with norms adjusted for age and education (Folstein et al., J. Psych. Res., 12 (1975), 196-198, Anthony et al., Psychological Med., 12 (1982), 397-408; Cockrell et al., Psychopharmacology, 24 (1988), 689-692; Crum et al., J. Am. Med. Assoc'n. 18 (1993), 2386-2391). The MMSE is a brief, quantitative measure of cognitive status in adults. It can be used to screen for cognitive decline or impairment, to estimate the severity of cognitive decline or impairment at a given point in time, to follow the course of cognitive changes in an individual over time, and to document an individual's response to treatment. Another suitable test is the Alzheimer Disease Assessment Scale (ADAS), in particular the cognitive element thereof (ADAS-cog) (See Rosen et al., Am. J. Psychiatry, 141 (1984), 1356-64).

For treating or preventing Alzheimer's disease, a suitable dosage level is about 0.01 to 250 mg/kg per day, preferably about 0.01 to 100 mg/kg per day, and more preferably about 0.05 to 50 mg/kg of body weight per day, of the active compound. The compounds may be administered on a regimen of 1 to 4 times per day. In some cases, however, a dosage outside these limits may be used.

The compound of formula I optionally may be administered in combination with one or more additional compounds known to be useful in the treatment or prevention of AD or the symptoms thereof. Such additional compounds thus include cognition-enhancing drugs such as acetylcholinesterase inhibitors (e.g. donepezil and galanthamine), NMDA antagonists (e.g. memantine) or PDE4 inhibitors (e.g. Ariflo™ and the classes of compounds disclosed in WO 03/018579, WO 01/46151, WO 02/074726 and WO 02/098878). Such additional compounds also include cholesterol-lowering drugs such as the statins, e.g. simvastatin. Such additional compounds similarly include compounds known to modify the production or processing of Aβ in the brain ("amyloid modifiers"), such as compounds which modulate the secretion of Aβ (including γ-secretase inhibitors, γ-secretase modulators and β-secretase inhibitors), compounds which inhibit the aggregation of Aβ, and antibodies which selectively bind to Aβ. Such additional compounds further include growth hormone secretagogues, e.g. as described in WO 2004/080459.

In this embodiment of the invention, the amyloid modifier may be a compound which inhibits the secretion of Aβ, for example an inhibitor of γ-secretase (such as those disclosed in WO 01/90084, WO 02/30912, WO 01/70677, WO 03/013506, WO 02/36555, WO 03/093252, WO 03/093264, WO 03/093251, WO 03/093253, WO 2004/039800, WO 2004/039370, WO 2005/030731, WO 2005/014553, WO 2004/089911, WO 02/081435, WO 02/081433, WO 03/018543, WO 2004/031137, WO 2004/031139, WO 2004/031138, WO 2004/101538, WO 2004/101539 and WO 02/47671), or a β-secretase inhibitor (such as those disclosed in WO 03/037325, WO 03/030886, WO 03/006013, WO 03/006021, WO 03/006423, WO 03/006453, WO 02/002122, WO 01/70672, WO 02/02505, WO 02/02506, WO 02/02512, WO 02/02520, WO 02/098849 and WO 02/100820), or any other compound which inhibits the formation or release of Aβ including those disclosed in WO 98/28268, WO 02/47671, WO 99/67221, WO 01/34639, WO 01/34571, WO 00/07995, WO 00/38618, WO 01/92235, WO 01/77086, WO 01/74784, WO 01/74796, WO 01/74783, WO 01/60826, WO 01/19797, WO 01/27108, WO 01/27091, WO 00/50391, WO 02/057252, US 2002/0025955 and US2002/0022621, and also including GSK-3 inhibitors, particularly GSK-3α inhibitors, such as lithium, as disclosed in Phiel et al, Nature, 423 (2003), 435-9.

Alternatively, the amyloid modifier may be a compound which modulates the action of γ-secretase so as to selectively attenuate the production of Aβ(1-42). Compounds reported to show this effect include certain non-steroidal antiinflammatory drugs (NSAIDs) and their analogues (see WO 01/78721 and US 2002/0128319 and Weggen et al Nature, 414 (2001) 212-16; Morihara et al; J. Neurochem., 83 (2002), 1009-12; and Takahashi et al, J. Biol. Chem., 278 (2003), 18644-70), and compounds which modulate the activity of PPARα and/or PPARδ (WO 02/100836). Further examples of γ-secretase modulators are disclosed in WO 2005/054193, WO 2005/013985, WO 2005/108362, WO 2006/008558 and WO 2006/043064..

Alternatively, the amyloid modifier may be a compound which inhibits the aggregation of Aβ or otherwise attenuates is neurotoxicicity. Suitable examples include chelating agents such as clioquinol (Gouras and Beal, Neuron, 30 (2001), 641-2) and the compounds disclosed in WO 99/16741, in particular that known as DP-109 (Kalendarev et al, J. Pharm. Biomed. Anal., 24 (2001), 967-75). Other inhibitors of Aβ aggregation suitable for use in the invention include the compounds disclosed in WO 96/28471, WO 98/08868 and WO 00/052048, including the compound known as Apan™ (Praecis); WO 00/064420, WO 03/017994, WO 99/59571 (in particular 3-aminopropane-1-sulfonic acid, also known as tramiprosate or Alzhemed™); WO 00/149281 and the compositions known as PTI-777 and PTI-00703 (ProteoTech); WO 96/39834, WO 01/83425, WO 01/55093, WO 00/76988, WO 00/76987, WO 00/76969, WO 00/76489, WO 97/26919, WO 97/16194, and WO 97/16191. Further examples include phytic acid derivatives as disclosed in US 4,847,082 and inositol derivatives as taught in US 2004/0204387.

Alternatively, the amyloid modifier may be an antibody which binds selectively to Aβ. Said antibody may be polyclonal or monoclonal, but is preferably monoclonal, and is preferably human or humanized. Preferably, the antibody is capable of sequestering soluble Aβ from biological fluids, as described in WO 03/016466, WO 03/016467, WO 03/015691 and WO 01/62801. Suitable antibodies include humanized antibody 266 (described in WO 01/62801) and the modified version thereof described in WO 03/016466. Suitable antibodies also include those specific to Aβ-derived diffusible ligands (ADDLS), as disclosed in WO 2004/031400.

As used herein, the expression "in combination with" requires that therapeutically effective amounts of both the compound of formula I and the additional compound are administered to the subject, but places no restriction on the manner in which this is achieved. Thus, the two species may be combined in a single dosage form for simultaneous administration to the subject, or may be provided in separate dosage forms for simultaneous or sequential administration to the subject. Sequential administration may be close in time or remote in time, e.g. one species administered in the morning and the other in the evening. The separate species may be administered at the same frequency or at different frequencies, e.g. one species once a day and the other two or more times a day. The separate species may be administered by the same route or by different routes, e.g. one species orally and the other parenterally, although oral administration of both species is preferred, where possible. When the additional compound is an antibody, it will typically be administered parenterally and separately from the compound of formula I.

### EXAMPLES

### MARK 3 Assay

MARK3 activity was assayed in vitro using a Cdc25C biotinylated peptide substrate (Cell Signalling Technologies). The phosphopeptide product was quantitated using a Homogenous Time-Resolved Fluorescence (HTRF) assay system (Park et al., 1999, Anal. Biochem. 269:94-104). The reaction mixture contained 50 mM HEPES/Tris-HCl, pH 7.4; 10 mM NaCl, 5 mM MgCl₂, 0.2 mM NaVO₄, 5 mM β-glycerol phosphate, 0.1% Tween-20, 2 mM dithiothreitol, 0.1% BSA, 10 µM ATP, 1 µM peptide substrate, and 10 nM recombinant MARK3 enzyme (University of Dundee) in a final volume of 12 µl. The buffer additionally contained protease inhibitor cocktail (Roche EDTA-free, 1 tab per 50 ml). The kinase reaction was incubated for 2 hours at 25°C, and then terminated with 3 µl Stop/Detection Buffer (50 mM HEPES, pH 7.0, 16.6 mM EDTA, 0.5M KF, 0.1% Tween-20, 0.1 % BSA, 2 µg/ml SLX^{ent} 665 (CISBIO), and 2 µg/ml Eu³⁺ cryptate label antibody (CISBIO)). The reaction was allowed to equilibrate overnight at 0°C, and relative fluorescent units were read on an HTRF enabled plate reader (e.g. TECAN GENios Pro).

Inhibitor compounds were assayed in the reaction described above to determine compound IC50s. Aliquots of compound dissolved in DMSO were added to the reaction wells in a third-log dilution series covering a range of 1 nM to 10 µM. Relative phospho substrate formation, read as HTRF fluorescence units, was measured over the range of compound concentrations and a titration curve generated.

The compounds listed below gave IC50 values of 1 µM or less, typically 500nM or less, and in preferred cases 50 nM less, in the above assay.

### Intermediate A

### Step 1 : 3-(Dimethylamino)-2-(4-hydroxynhenyl)acrylaldehyde.

To DMF (freshly distilled over phthalic anhydride, 66 mL)at 0°C, POCl₃ (25.39 g, 0.166 mol) was added dropwise under stirring and cooling. The reaction mixture was stirred at room temperature for 10 min, then 4-hydroxyphenylacetic acid (8.40 g, 0.055 mol) was added, and the mixture was stirred at 80-85°C for 6 h. After cooling, the mixture was poured on 180 g of ice, and 20 g of NaOH and then 115 mL of 10M NaOH solution were added slowly, while maintaining the temperature below 40°C. The mixture was then stirred at room temperature for 2 h and slowly neutralized with concentrated HCl to pH 3. The precipitate was filtered off, washed with water and dried in vacuum at 50°C for 20 h. to afford the desired aldehyde. Yield: 5.51 g (52.2%).

### Step 2 :4-(3-Bromopyrazolo[1,5-a]pyrimidin-6-yl)phenol

A mixture of 3-(dimethylamino)-2-(4-hydroxyphenyl)acrylaldehyde (5.00 g, 0.026 mol), 3-amino-4-bromopyrazole (4.23 g, 0.026 mol), 84 mL of ethanol and 4.2 mL of acetic acid was refluxed for 12 h. The mixture was cooled and the precipitate filtered off, washed twice with water and a small amount of ethanol, then dried in vacuum at 40-50°C for 8 h. to give the desired phenol. Yield: 4.66 g (61.5%).

### Step 3-Bromo-6-[4-(2-piperidin-1-ylethoxy)phenyl]pyrazolo[1,5-a]pyrimidine (Intermediate A).

To a solution of the phenol of Step 2 (4.6 g, 0.016 mol) in 65 mL of absolute DMF, were added N-(2-chloroethyl)piperidine (3.83 g, 0.021 mol), Cs₂CO₃ (13.55 g, 0.042 mol) and NaI (0.285 g, 0.0019 mol) in this order. The reaction mixture was stirred at 60-65°C for 18 h., cooled down and poured into 300 mL of 50% solution of NaOH, The product was extracted with ethyl acetate (3x150 mL). The combined organic extracts were twice washed with brine (2x200 mL), dried over anhydrous MgSO₄, concentrated on a rotary evaporator and evaporated with xylene twice. The residue was subjected to chromatography on silica gel, eluting with chloroform-methanol, 15:1 to afford desired Intermediate A. Yield: 4.33 g (67.4%).

### Example 1 : Methyl 4-{6-[4-(2-piperidin-1-ylethoxy)phenyl]pyrazolo[1,5-a]pyrimidin-3-yl}thiophene-2-carbozylate

### Step 1 : methyl 4-(4,4,5,5-tetramethyl-1,3,2-dioxaborolan-2-yl)thiophene-2-carboxylate

4-Bromothiophene-2-carboxylic acid (0.418 g, 2 mmol) was dissolved in methanol (1 mL), and concentrated sulfuric acid (0.039 g, 0.4 mmol) was added. The mixture was refluxed for 10 h, poured into water, and subjected to 3-fold extraction with ethyl acetate. The organic layer was washed with K₂CO₃solution, concentrated, dried over MgSO₄, filtered and evaporated to give methyl 4-bromothiophene-2-carboxylate, weight 0.4 g (90% yield).

A flask containing PdCl₂(dppf) (0.32 g, 0.43 mmol), dppf (0.24 g, 0.43 mmol), KOAc (4.23 g, 0.043 mol), and pinacolediborone (5.5 g, 0.021 mol) was flushed with argon, then a solution of the ester from the foregoing step (3.2 g, 0.014 mol) in dioxane (60 mL) was added. The mixture was stirred at 85°C under argon atmosphere for 40 h. Water (5-fold excess) was added, and the mixture was subjected to 3-fold extraction with ethyl acetate. The organic layer was washed with brine, concentrated, dried over MgSO₄, filtered, and evaporated to give the crude methyl 4-(4,4,5,5-tetramethyl-1,3,2-dioxaborolan-2-yl)thiophene-2-carboxylate (5.1 g, purity 85% according to ¹H NMR data). This crude boronate was used without further purification.

### Step 2

Dioxane (45 mL) and 1M Na₂CO₃-solution (10.4 mL) were added to a mixture of Intermediate A (2.15 g, 5.36 mmol) and methyl 4-(4,4,5,5-tetramethyl-1,3,2-dioxaborolan-2-yl)thiophen-2-carboxylate (2.20 g of the crude boronate, 6.97 mmol). The mixture was flushed with argon, then Pd(Ph₃P)₄ (0.31 g, 0.27 mmol) was added, the temperature was elevated to 85°C, and stirring was continued at this temperature for 16 h. After cooling to room temperature, the reaction mixture was poured into water (5-fold excess) and subjected to 3-fold extraction with chloroform. The organic layer was washed with brine, concentrated, dried over MgSO_{4,} filtered, and the solvent was evaporated. The residue was purified by column chromatography on silica gel (chloroform/methanol, 15:1) to give the desired ester, weight 2.22 g (90% yield).
m/z [MH⁺] 463.

### Example 2 : 4-{6-[4-(2-Piperidin-1-ylethoxy)phenyl]pyrazolo[1,5-a]pyrimidin-3-yl}-N-(2,2,2-trifluoroethyl)thiophene-2-carboxamide

A suspension of the methyl ester from Example 1 (2.22 g, 4.82 mmol) in 15 mL of 9M aqueous HCl was refluxed for 24 h. Water was evaporated, and the residue was re-evaporated with acetonitrile. The solid residue (hydrochloride) was transferred on to a Shott filter, washed with ether, and dried in a vacuum drying oven to afford 2.0 g (96% yield) of the desired carboxylic acid.

BOP (0.16 g, 0.36 mmol) was added to a suspension of the acid from the foregoing step (0.15 g, 0.33 mmol) in dry DMF (10 mL). The mixture was stirred for 20 min., then diisopropylethylamine (0.3 mL, 1.65 mmol) and trifluoroethylamine (0.065 g, 0.66 mmol) were added, and the mixture was stirred at room temperature overnight. Water (10-fold excess) was added, the resulting precipitate was filtered, washed with 5% NaHCO₃, water, ether, and dried in a vacuum drying oven to afford 0.075 g (44% yield) of the desired 4-{6-[4-(2-piperidin-1-ylethoxy)phenyl]pyrazolo[1,5-a]pyrimidin-3-yl}-N-(2,2,2-trifluoroethyl)thiophene-2-carboxamide. m/z [MH⁺] 530.

### Example 3 : Methyl-4-(6-{4-[2-(dimethylamino)ethoxy]phenyl}pyrazolo[1,5-a]pyrimidin-3-yl) thiophene-2-carboxylate

### Step 1 : 2-[4-(3-bromopyrazolo[1,5-a]pyrimidin-6-yl)phenoxy]-N,N-dimethylethanamine

A mixture of the phenol from Step 2 of Intermediate A (0.406 g, 1.40 mmol), 2-(dimethylamine)ethyl chloride hydrochloride (0.282 g, 1.96 mmol), Cs₂CO₃ (1.27 g, 3.92 mmol), NaI (0.025 g, 0.168 mol) and dry DMF (12 mL) was stirred at 60°C for 16 h. After cooling to room temperature, water (50 mL) was added, the resulting precipitate was separated by filtration, washed with water, and re-evaporated with chloroform. The residue was purified by column chromatography on silica gel (chloroform/NH₃-saturated methanol, 20:1) to yield the desired 2-[4-(3-bromopyrazolo[1,5-a]pyrimidin-6-yl)phenoxy]-N,N-dimethylethanamine (0.3 g 60% yield).

### Step 2

The Suzuki reaction was carried out as described in Example 1 Step 2 with the use of 2-[4-(3-bromopyazalo[1,5-a]pyrimidin-6-yl)phenoxy]-N,N-dimethylethanamine (0.3 g, 0.3 mmol), methyl 4-(4,4,5,5-tetramethyl-1,3,2-dioxaborolan-2-yl)thiophene-2-carboxylate (0.35 g of the crude boronate, 1.08 mmol), 1M Na₂CO₃-solution (1.6 mL), Pd(Ph₃P)₄ (0.048 g, 0.04 mmol), and dioxane (8 mL). The reaction mixture was stirred at 85°C for 16 h, cooled to room temperature, then water (5-fold excess) was added. After 3-fold extraction with chloroform, the organic layer was washed with brine, concentrated, dried over MgSO₄, filtered, and the solvent was evaporated. The residue was purified by column chromatography on silica gel (chloroform/NH₃-saturated methanol 20:1) to afford the desired ester, 0.23 g (65% yield).

### Example 4 : 4-(6-{4-[2-(dimethylamino)ethoxy]phenyl}pyrazolo[1,5-a]pyrimidin-3-yl)-N-(2,2,2-trifluoroethyl)thiophene-2-carboxamide

Prepared from the product of Example 3 by the procedure of Example 2.
¹H NMR (400 MHz, DMSO-*d₆*): 2.23-2.24 (6H, m), 2.63-2.67 (2H, t, J=5.87 Hz, J=5.87 Hz), 4.08-4.15 (4H, m), 7.09-7.13 (2H, d, J=8.81 Hz), 7.79-7.82 (2H, d, J=8.80 Hz), 8.22-8.24 (1H, d, J=1.24 Hz), 8.50-8.51(1H, d, J=1.23 Hz), 8.61 (1H, s), 9.02-9.03 (1H, d, J=2.20 Hz), 9.20-9.24 (1H, t, J=6.36 Hz, J=6.11 Hz), 9.41-9.43 (1H, d, J=2.20 Hz).
LC-MS APCI: *m*/*z* 490.1 [M + H]⁺.

### Example 5 : Representative Procedure

To a solution of the appropriate boronic acid/ester (260 µmol, 1.3 eq.) in dioxane (500 uL) in a glove box under an atmosphere of nitrogen, were added a solution of Na₂C0₃ (300 µmol, 1.5 eq.) in water (500 µL) and a solution of 3-bromo-6-(4-methoxyphenyl)pyrazolo[1,5-a]pyrimidine (WO 98/54093) (200 µmol, 1 eq.) in dioxane (500 µL). Then, under an argon atmosphere, a solution of Pd(PPh₃)₄ (12 mg) in dioxane (400 µL) was added The mixtures were kept at 85°C overnight, cooled and evaporated. The residue was taken up in dichloromethane (2 mL) and H₂O (500 µL). The organic layer was separated, evaporated and the products were purified by preparative HPLC.

### Examples 6-81

The following were prepared by methods analogous to those described above:

| **Example.** | **Structure** | **m/z [MH+]** |
|---|---|---|
| **Example 6** | 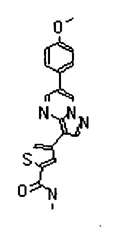 | **365** |
| **Example 7** | 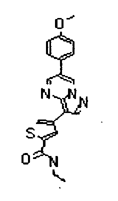 | **379** |
| **Example 8** | 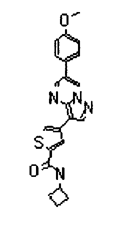 | **405** |
| **Example 9** | 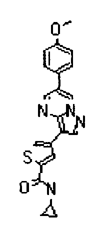 | **391** |
| **Example 10** | 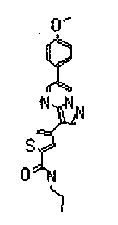 | **393** |
| **Example 11** | 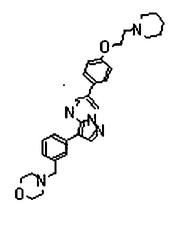 | **498** |
| **Example 12** | 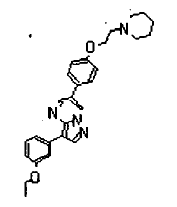 | **443** |
| **Example 13** | 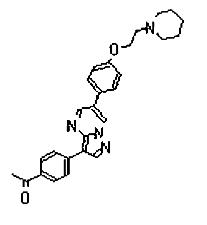 | **441** |
| **Example 14** | 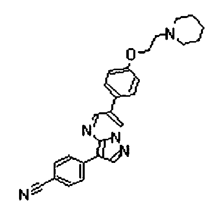 | **424** |
| **Example 15** | 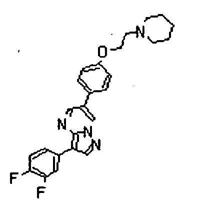 | **435** |
| **Example 16** | 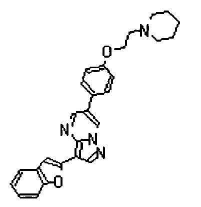 | **439** |
| **Example 17** | 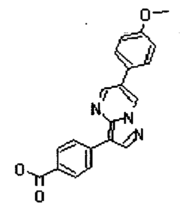 | **346** |
| **Example 18** | 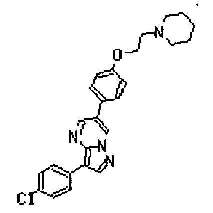 | **433** |
| **Example 19** | 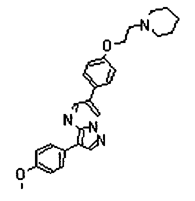 | **429** |
| **Example 20** | 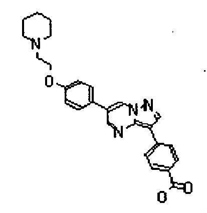 | **443** |
| **Example 21** | 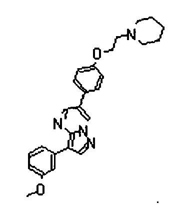 | **429** |
| **Example 22** | 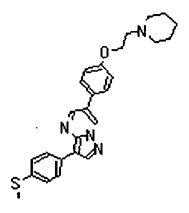 | **445** |
| **Example 23** | 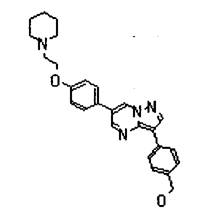 | **429** |
| **Example 24** | 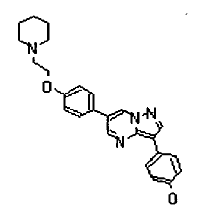 | **415** |
| **Example 25** | 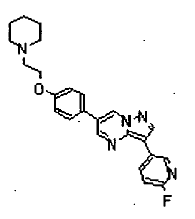 | **418** |
| **Example 26** | 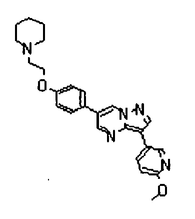 | **430** |
| **Example 27** | 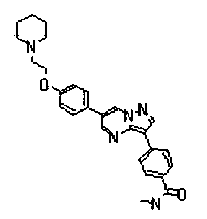 | **456** |
| **Example 28** | 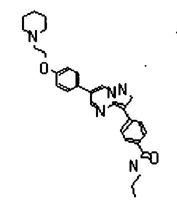 | **484** |
| **Example 29** | 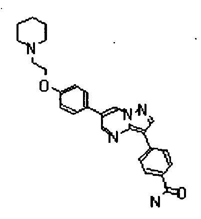 | **442** |
| **Example 30** | 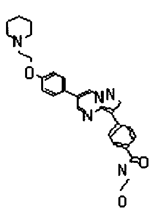 | **486** |
| **Example 31** | 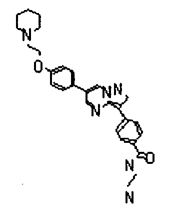 | **485** |
| **Example 32** | 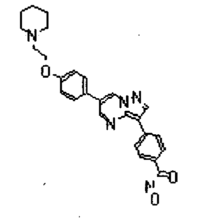 | **458** |
| **Example 33** | 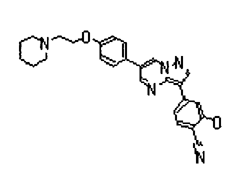 | **440** |
| **Example 34** | 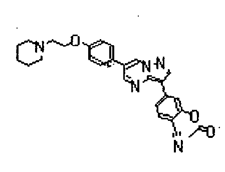 | **482** |
| **Example 35** | 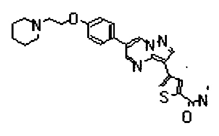 | **462** |
| **Example 36** | 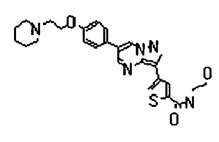 | **492** |
| **Example 37** | 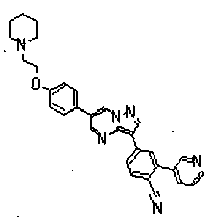 | **501** |
| **Example 38** | 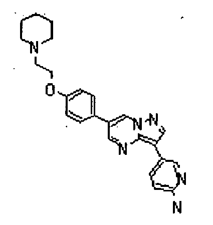 | **415** |
| **Example 39** | 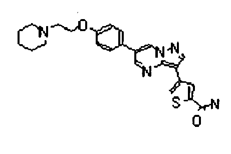 | **448** |
| **Example 40** | 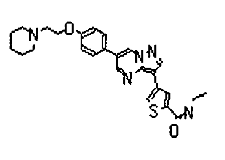 | **476** |
| **Example 41** | 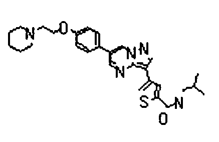 | **504** |
| **Example 42** | 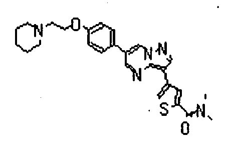 | **476** |
| **Example 43** | 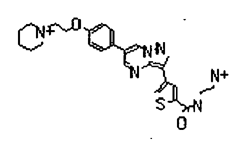 | **491** |
| **Example 44** | 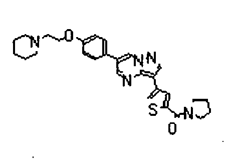 | **502** |
| **Example 45** | 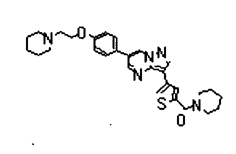 | **516** |
| **Example 46** | 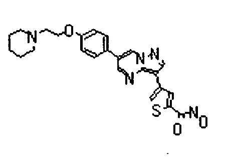 | **464** |
| **Example 47** | 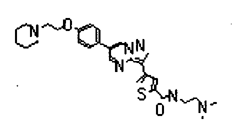 | **519** |
| **Example 48** | 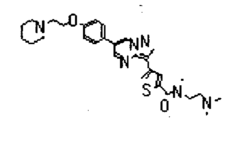 | **533** |
| **Example 49** | 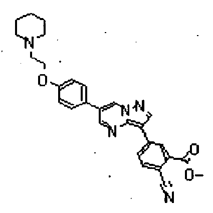 | **468** |
| **Example 50** | 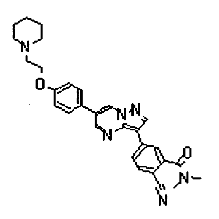 | **495** |
| **Example 51** | 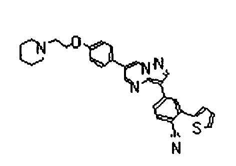 | **506** |
| **Example 52** | 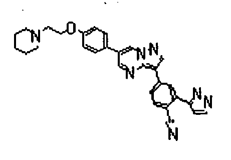 | **490** |
| **Example 53** | 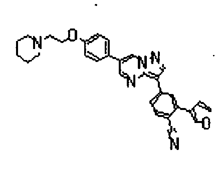 | **490** |
| **Example 54** | 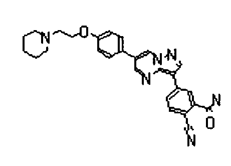 | **467** |
| **Example 55** | 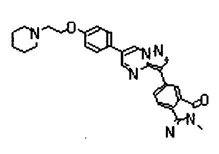 | **481** |
| **Example 56** | 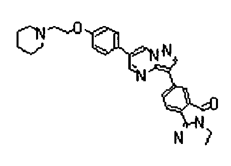 | **495** |
| **Example 57** | 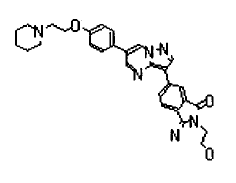 | **511** |
| **Example 58** | 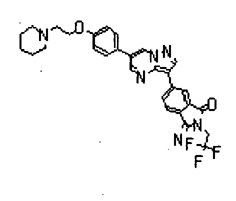 | **549** |
| **Example 59** | 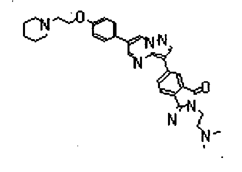 | **538** |
| **Example 60** | 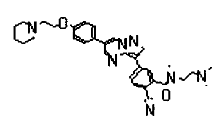 | **552** |
| **Example 61** | 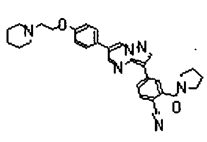 | **521** |
| **Example 62** | 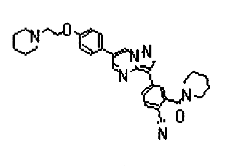 | **535** |
| **Example 63** | 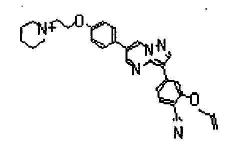 | **480** |
| **Example 64** | 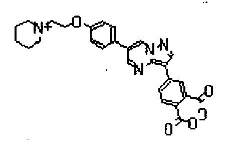 | **487** |
| **Example 65** | 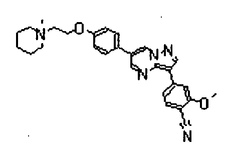 | **469** |
| **Example 66** | 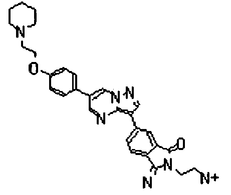 | **510** |
| **Example 67** | 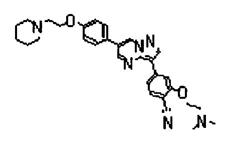 | **511** |
| **Example 68** | 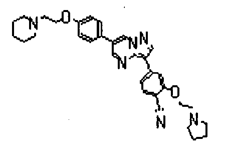 | **537** |
| **Example 69** | 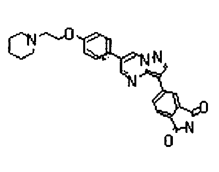 | **468** |
| **Example 70** | 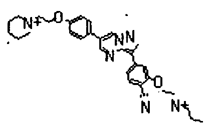 | **525** |
| **Example 71** | 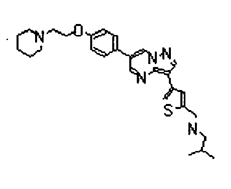 | **490** |
| **Example 72** | 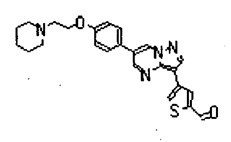 | **433** |
| **Example 73** | 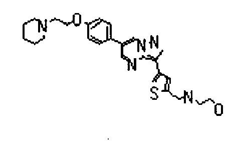 | **476** |
| **Example 74** | 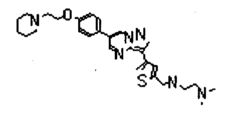 | **505** |
| **Example 75** | 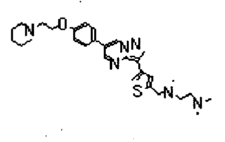 | **519** |
| **Example 76** | 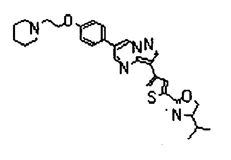 | **516** |
| **Example 77** | 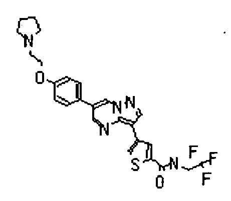 | **516** |
| **Example 78** | 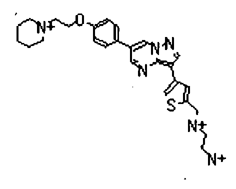 | **477** |
| **Example 79** | 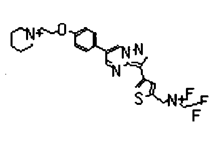 | **516** |
| **Example 80** | 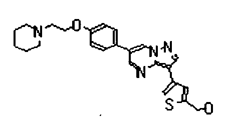 | **435** |
| **Example 81** | 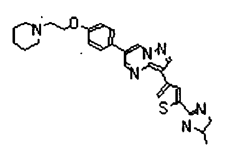 | **487** |

In the above table, hydrogen atoms are to be inferred where heteroatoms are shown with one or more unsatisfied valencies.
Also prepared by similar routes were the following:

| **Example** | **Structure** | **m/z (MH⁺)** |
|---|---|---|
| 82 | 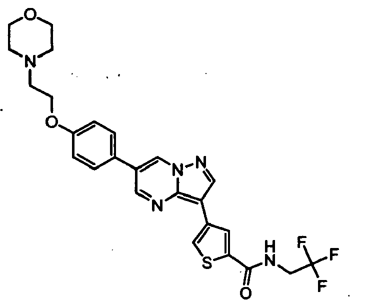 | 531.56 |
| 83 | 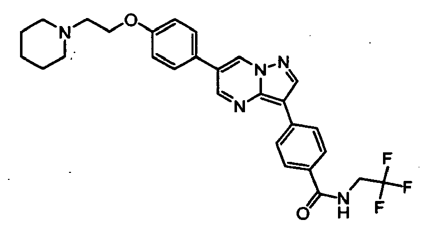 | 523.56 |
| 84 | 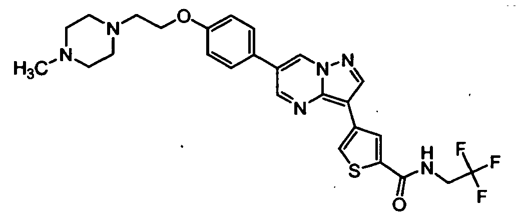 | 544.60 |
| 85 | 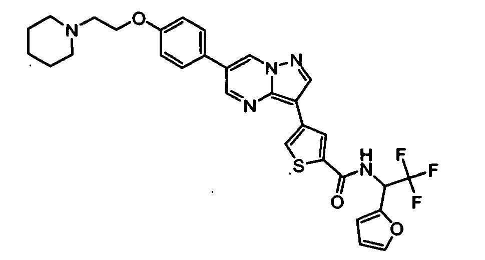 | 595.65 |
| 86 | 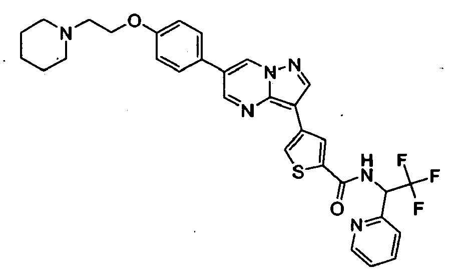 | 606.675 |
| 87 | 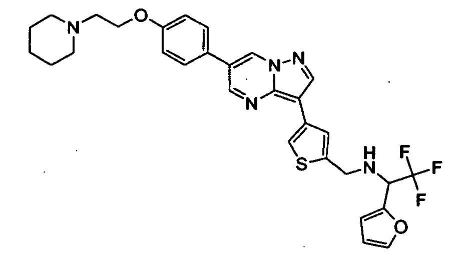 | 581.665 |
| 88 | 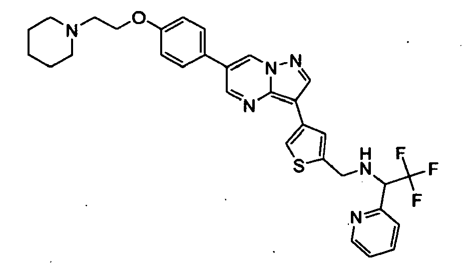 | 592.692 |
| 89 | 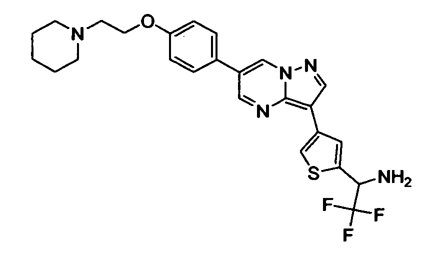 | 501.578 |
| 90 | 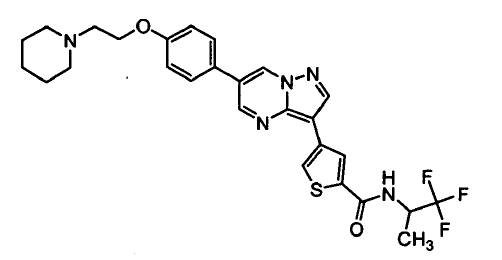 | 543.616 |
| 91 | 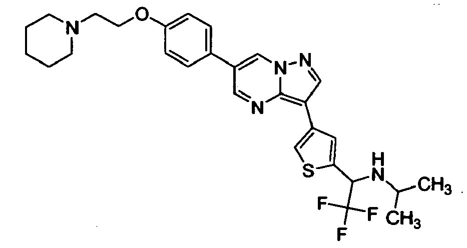 | 543.659 |
| 92 | 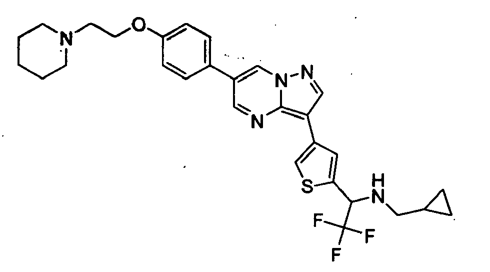 | 555.670 |
| 93 | 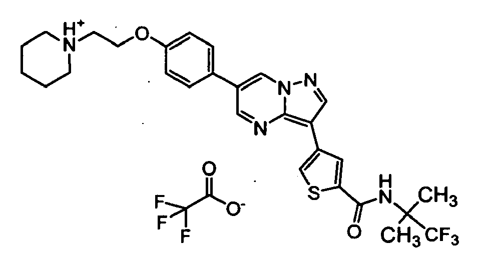 | 557.643 |
| 94 | 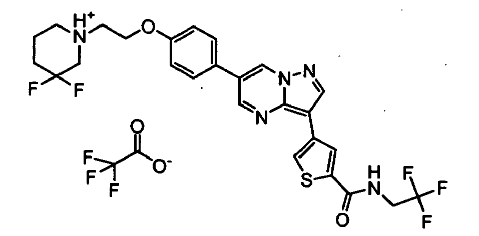 | 565.569 |
| 95 | 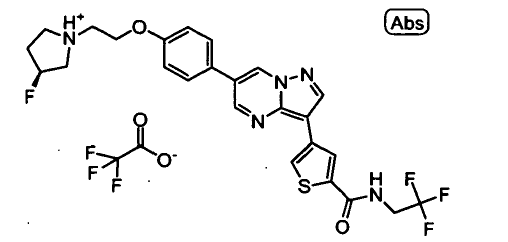 | 533.552 |
| 96 | 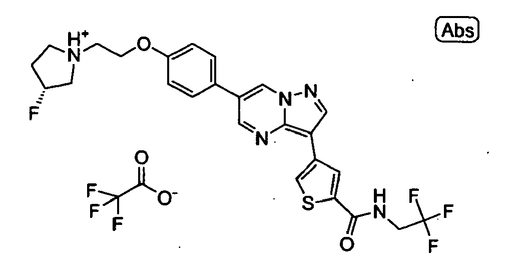 | 533.552 |
| 97 | 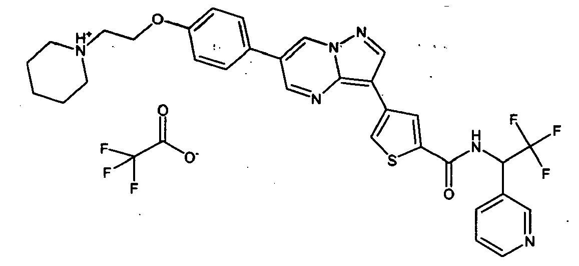 | 606.675 |
| 98 | 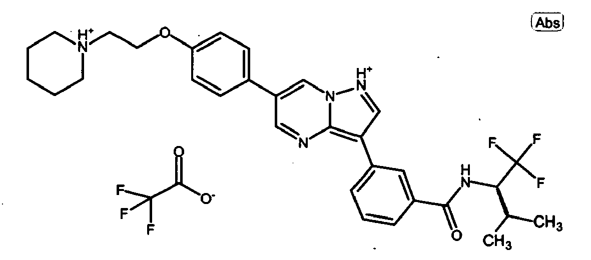 | 565.644 |
| 99 | 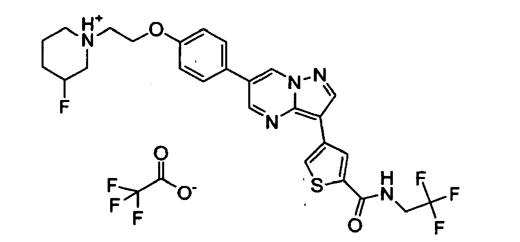 | 547.579 |
| 100 | 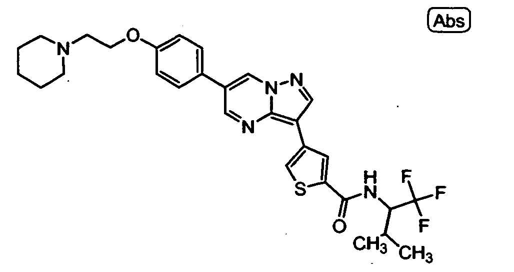 | 571.670 |
| 101 | 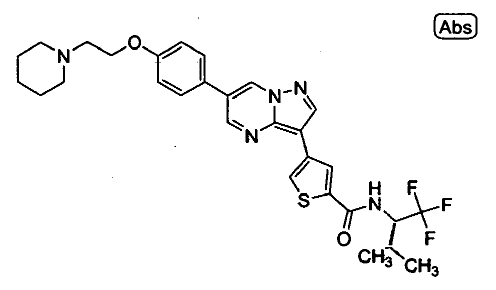 | 571.670 |
| 102 | 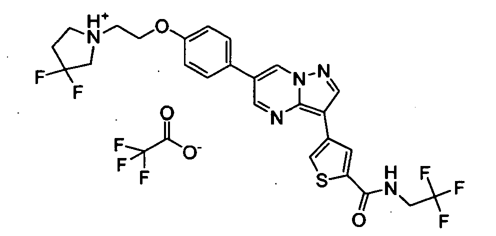 | 551.542 |
| 103 | 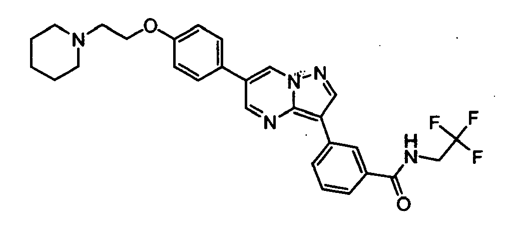 | 523.563 |
| 104 | 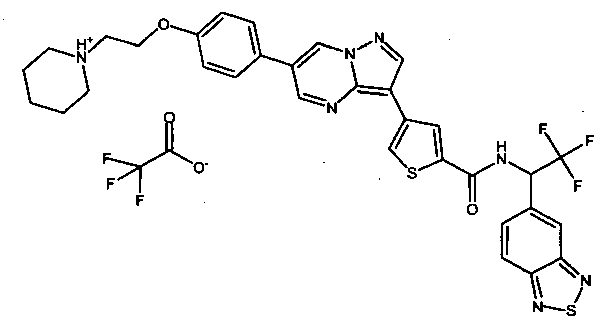 | 663.749 |
| 105 | 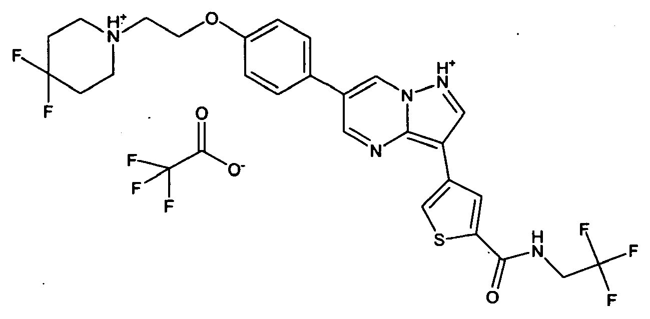 | 565.569 |
| 106 | 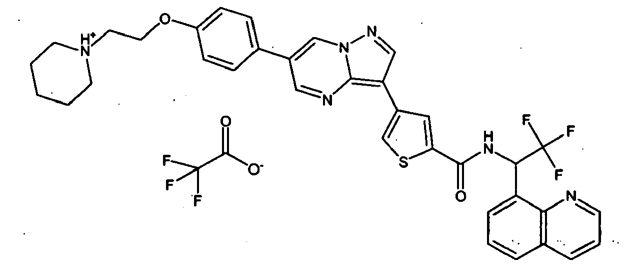 | 656.736 |
| 107 | 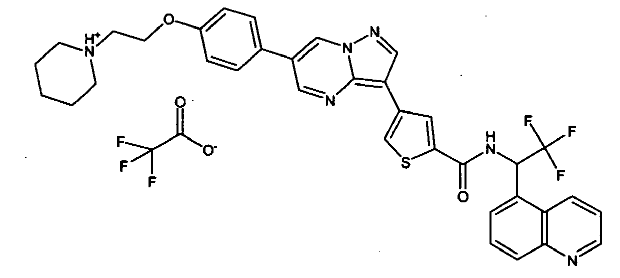 | 656.736 |
| 108 | 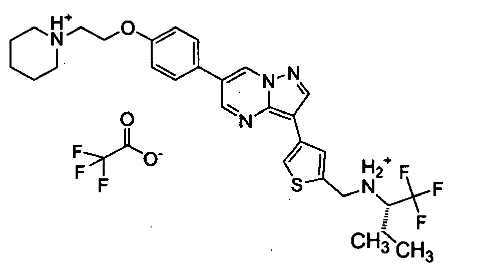 | 557.686 |
| 109 | 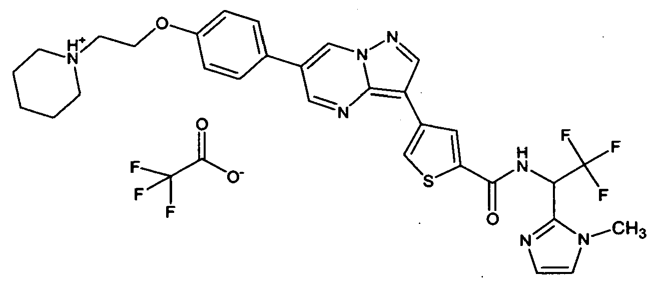 | 609.679 |
| 110 | 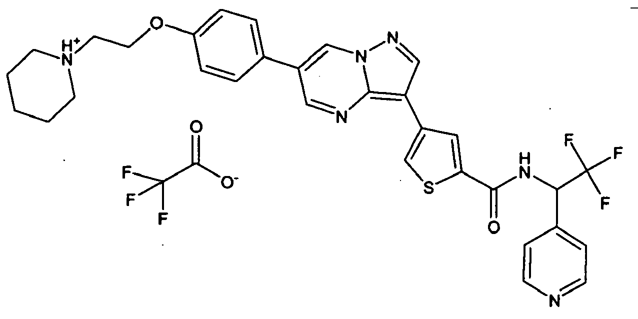 | 606.675 |
| 111 | 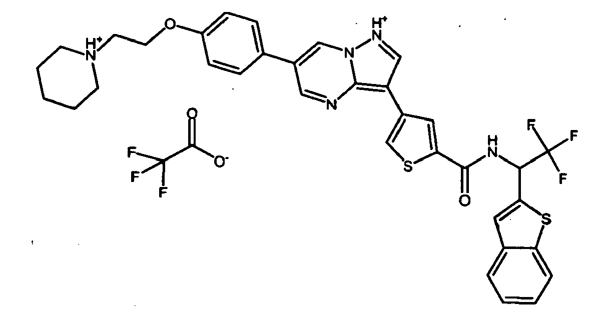 | 661.774 |
| 112 | 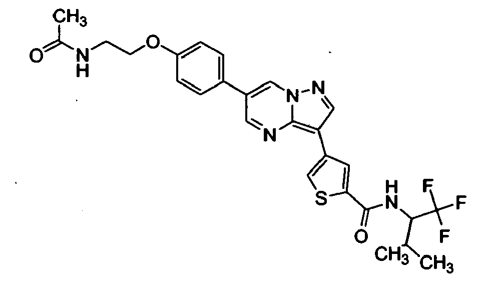 | 545.588 |
| 113 | 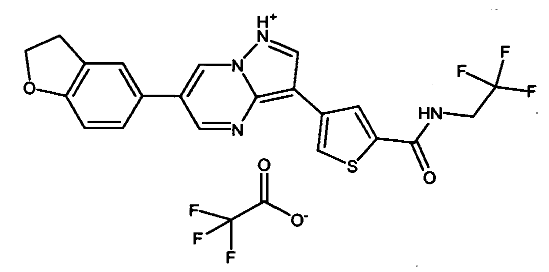 | 444.438 |
| 114 | 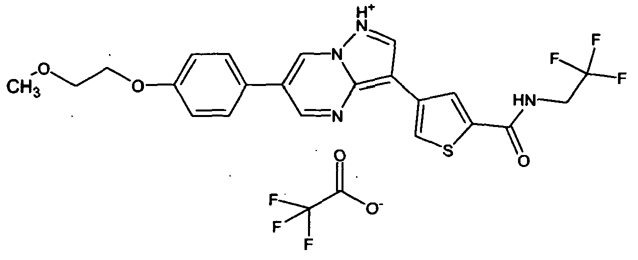 | 476.481 |
| 115 | 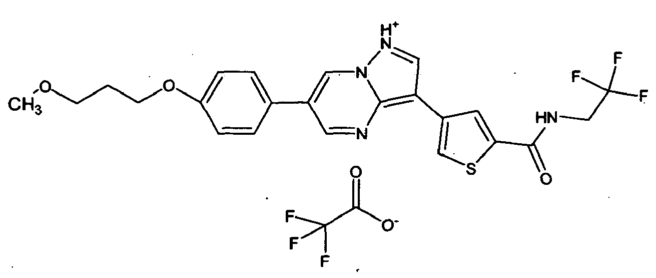 | 490.508 |

## Claims

1. The use, for the manufacture of a medicament for treatment or prevention of a neurodegenerative disease associated with hyperphosphorylation of tau, of a compound according to formula I: 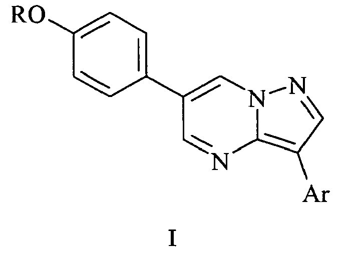 or a pharmaceutically acceptable salt or hydrate thereof; wherein:
R represents C₁₋₄alkyl which is optionally substituted with halogen, CN, CF₃, OR¹, NR¹R², NHPh or NHCOC₁₋₄alkyl; or R may complete a fused tetrahydrofuran ring;
Ar represents phenyl or optionally benzofused 5- or 6-membered heteroaryl, any of which optionally bears up to 3 independently-selected R³ substituents;
R¹ and R² independently represent H or C₁₋₄alkyl, or R¹ and R² bonded to the same nitrogen atom may complete a heterocyclic ring of up to 6 members which optionally comprises one additional heteroatom selected from N, O and S and which optionally bears up to 2 substituents selected from C₁₋₄alkyl, CN, CF₃, halogen and oxo;
R³ represents halogen, CN, R⁵, SR⁵, X-OR⁴, X-N(R⁴)₂, CH(CF₃)-N(R⁴)₂, COR⁴, CONHOH, phenyl, 5- or 6-membered heteroaryl or C-heterocyclyl, said phenyl, 5- or 6-membered heteroaryl or C-heterocyclyl optionally bearing up to 2 substituents selected from C₁₋₄alkyl, CF₃ and halogen; or when Ar represents phenyl two R³ groups attached to adjacent ring atoms on Ar may complete a fused 5- or 6-membered carbocyclic or heterocyclic ring which optionally bears up to 3 substituents selected from oxo, imino, and R⁵;
R⁴ represents H, CF₃, CH(CF₃)-Ar¹, or alkyl, alkenyl, cycloalkyl or cycloalkylalkyl of up to 6 carbon atoms which is optionally substituted with halogen, CN, CF₃, OR¹ or NR¹R²; or two R⁴ groups bonded to the same nitrogen atom may complete a heterocyclic ring of up to 6 members which optionally comprises one additional heteroatom selected from N, O and S and which optionally bears up to 2 substituents selected from C₁₋₄alkyl, CF₃, halogen and oxo;
R⁵ represents R⁴ that is not H;
Ar¹ represents an aromatic mono- or bicyclic ring system of up to 10 ring atoms of which 0-3 are selected from N, O and S and the rest are carbon, said ring system bearing 0-3 substituents selected from halogen, CF₃ and C₁₋₄alkyl;
X represents a bond, CH₂ or CO; and
"C-heterocyclyl" refers to nonaromatic heterocyclic rings of 5 or 6 ring atoms, up to 2 of which are selected from N, O and S, said ring being attached via a ring carbon atom;
wherein said neurodegenerative disease associated with hyperphosphorylation of tau is selected from Alzheimer's disease (AD), frontotemporal dementia, Pick's disease and parkinsonism linked to chromosome 17 (FTDP-17).

2. A compound of formula I as defined in claim 1, or a pharmaceutically acceptable salt or hydrate thereof for use in treatment or prevention of a neurodegenerative disease associated with hyperphosphorylation of tau in a human patient, wherein said neurodegenerative disease associated with hyperphosphorylation of tau is selected from Alzheimer's disease (AD), frontotemporal dementia, Pick's disease and parkinsonism linked to chromosome 17 (FTDP-17).

3. Use according to claim 1 wherein said compound is a compound of formula II: 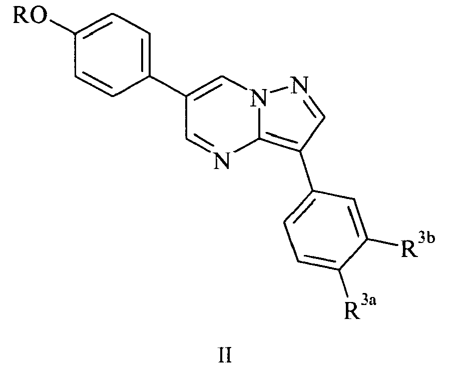 or a pharmaceutically acceptable salt or hydrate thereof; wherein R^{3a} and R^{3b} independently represent H or R³, and R and R³ are as defined in claim 1.

4. Use according to claim 1 wherein said compound is a compound formula III: 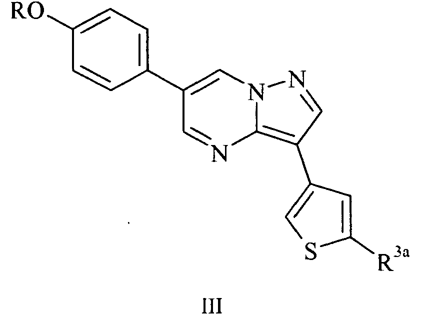 or a pharmaceutically acceptable salt or hydrate thereof; wherein R^{3a} represents H or R³, and R and R³ are as defined in claim 1.

5. Use according to any of claims 1, 3 and 4 wherein R represents CH₂CH₂NR¹R² or CH₂CH₂CH₂NR¹R², where R¹ and R² are as defined in claim 1.

6. A compound of formula III: 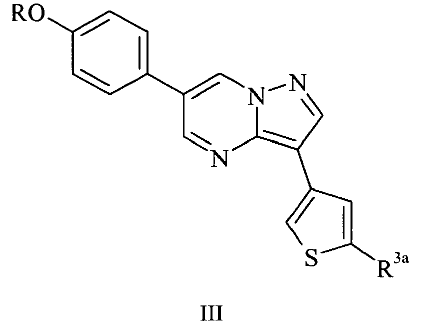 or a pharmaceutically acceptable salt or hydrate thereof; wherein
R represents C₁₋₄alkyl which is substituted with halogen, CN, CF₃, OR¹, NR¹R², NHPh or NHCOC₁₋₄alkyl;
R^{3a} represents R³;
and R¹, R² and R³ are as defined in claim 1.

7. A compound according to claim 6 wherein R represents CH₂CH₂NR¹R² or CH₂CHCHNR¹R².

8. A compound according to claim 6 or claim 7 wherein R^{3a} represents CONHR⁴ where R⁴ is H or C₁₋₄ alkyl which is optionally substituted with CF₃, OR¹ or NR¹R².

9. A compound according to claim 6 wherein R^{3a} represents CH₂NHR⁴ or CONHR⁴ and R⁴ represents CH(CF₃)-Ar¹ where Ar¹ is as defined in claim 1.

10. A compound according to claim 6 wherein R and R^{3a} are as listed in the following table:
| **R** | **R^{3a}** |
|---|---|
| 2-(piperidin-1-yl)ethyl | CO₂Me |
| 2-(piperidin-1-yl)ethyl | CONHMe |
| 2-(piperidin-1-yl)ethyl | CONHCH₂CH₂OH |
| 2-(piperidin-1-yl)ethyl | CONH₂ |
| 2-(piperidin-1-yl)ethyl | CONHEt |
| 2-(piperidin-1-yl)ethyl | CONH-isobutyl |
| 2-(piperidin-1-yl)ethyl | CON(Me)₂ |
| 2-(piperidin-1-yl)ethyl | CONHCH₂CH₂NH₂ |
| 2-(piperidin-1-yl)ethyl | CO-(1-pyrrolidinyl) |
| 2-(piperidin-1-yl)ethyl | CO-(1-piperidinyl) |
| 2-(piperidin-1-yl)ethyl | CONHCH₂CF₃ |
| 2-(piperidin-1-yl)ethyl | CONHOH |
| 2-(piperidin-1-yl)ethyl | CONHCH₂CH₂N(Me)₂ |
| 2-(piperidin-1-yl)ethyl | CON(Me)CH₂CH₂N(Me)₂ |
| 2-(piperidin-1-yl)ethyl | CH₂NH-isobutyl |
| 2-(piperidin-1-yl)ethyl | CHO |
| 2-(piperidin-1-yl)ethyl | CH₂NHCH₂CH₂OH |
| 2-(piperidin-1-yl)ethyl | CH2NHCH₂CH₂N(Me)₂ |
| 2-(piperidin-1-yl)ethyl | CH₂N(Me)CH₂CH₂N(Me)₂ |
| 2-(piperidin-1-yl)ethyl | 4-isopropyl-4,5-dihydro-1,3-oxazol-2-yl |
| 2-(dimethylamino)ethyl | CH₂CF₃ |
| 2-(pyrrolidin-1-yl)ethyl | CH₂CF₃ |
| 2-(piperidin-1-yl)ethyl | CH₂NHCH₂CH₂NH₂ |
| 2-(piperidin-1-yl)ethyl | CH₂NHCH₂CF₃ |
| 2-(piperidin-1-yl)ethyl | CH₂OH |
| 2-(piperidin-1-yl)ethyl | 5-methyl-4,5-dihydro-1*H*-imidazol-2-yl |

11. A compound according to claim 6 wherein R and R^{3a} are as listed in the following table:
| **R** | **R^{3a}** |
|---|---|
| 2-(morpholin-4-yl)ethyl | CONHCH₂CF₃ |
| 2-(4-Me-piperazin-1-yl)ethyl | CONHCH₂CF₃ |
| 2-(piperidin-1-yl)ethyl | CONHCH(CF₃)-(2-furyl) |
| 2-(piperidin-1-yl)ethyl | CONHCH(CF₃)-(2-pyridyl) |
| 2-(piperidin-1-yl)ethyl | CH₂NHCH(CF₃)-(2-furyl) |
| 2-(piperidin-1-yl)ethyl | CH₂NHCH(CF₃)-(2-pyridyl) |
| 2-(piperidin-1-yl)ethyl | CH(CF₃)-NH2 |
| 2-(piperidin-1-yl)ethyl | CONHCH(CF₃)-Me |
| 2-(piperidin-1-yl)ethyl | CH(CF₃)-NH-isopropyl |
| 2-(piperidin-1-yl)ethyl | CH(CF₃)-NHCH₂cyclopropyl |
| 2-(piperidin-1-yl)ethyl | CONHC(Me)₂CF₃ |
| 2-(3,3-di-F-piperidin-1-yl)ethyl | CONHCH₂CF₃ |
| 2-(3-F-pyrrolidin-1-yl) | CONHCH₂CF₃ |
| 2-(piperidin-1-yl)ethyl | CONHCH(CF₃)-(3-pyridyl) |
| 2-(3-F-piperidin-1-yl)ethyl | CONHCH₂CF₃ |
| 2-(piperidin-1-yl)ethyl | CONHCH(CF₃)-isopropyl |
| 2-(3,3-di-F-pyrrolidin-1yl)ethyl | CONHCH₂CF₃ |
| 2-(4,4-di-F-piperidin-1yl)ethyl | CONHCH₂CF₃ |
| 2-(piperidin-1-yl)ethyl | CONHCH(CF₃)-(quinolin-5-yl) |
| 2-(piperidin-1-yl)ethyl | CONHCH(CF₃)-(quinolin-8-yl) |
| 2-(piperidin-1-yl)ethyl | CH₂NHCH(CF₃)-isopropyl |
| 2-(piperidin-1-yl)ethyl | CONHCH(CF₃)-(1-Me-imidazol-2-yl) |
| 2-(piperidin-1-yl)ethyl | CONHCH(CF₃)-(4-pyridyl) |
| 2-(piperidin-1-yl)ethyl | CONHCH(CF₃)-(benzthiophen-2-yl) |
| 2-AcNH-ethyl | CONHCH(CF₃)-isopropyl |
| Fused tetrahydrofuran | CONHCH₂CF₃ |
| 2-methoxyethyl | CONHCH₂CF₃ |
| 3-methoxypropyl | CONHCH₂CF₃ |

12. A pharmaceutical composition comprising a compound according to any of claims 6-11 and a pharmaceutically acceptable carrier.

13. A compound according to any of claims 6-11 for use in medicine.

## Patentansprüche

1. Die Verwendung einer Verbindung gemäß Formel I: 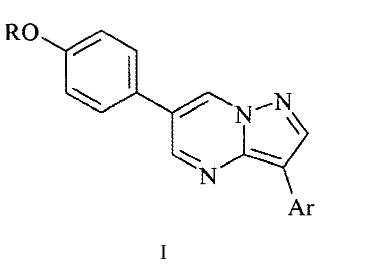 oder eines pharmazeutisch annehmbaren Salzes oder Hydrats davon zur Herstellung eines Medikaments zur Behandlung oder Prävention einer neurodegenerativen Erkrankung, die mit der Hyperphosphorylierung von Tau verbunden ist, wobei:
R C₁₋₄-Alkyl bedeutet, das gegebenenfalls substituiert ist mit Halogen, CN, CF₃, OR¹, NR¹R², NHPh oder NHCOC₁₋₄-Alkyl, oder R einen kondensierten Tetrahydrofuranring vervollständigen kann,
Ar Phenyl oder gegebenenfalls benzokondensiertes 5- oder 6-gliedrige Heteroaryl bedeutet, wobei jedes davon gegebenenfalls bis zu 3 unabhängig ausgewählte R³-Substituenten trägt,
R¹ und R² unabhängig H oder C₁₋₄-Alkyl bedeuten, oder R¹ und R², die an dasselbe Stickstoffatom gebunden sind, einen heterocyclischen Ring mit bis zu 6 Gliedern, der gegebenenfalls ein zusätzliches Heteroatom, ausgewählt aus N, O und S, umfasst und der gegebenenfalls bis zu 2 Substituenten, ausgewählt aus C₁₋₄-Alkyl, CN, CF₃, Halogen und Oxo, trägt, vervollständigen können,
R³ Halogen, CN, R⁵, SR⁵, X-OR⁴, X-N(R⁴)₂, CH(CF₃)-N(R⁴)₂, COR⁴, CONHOH, Phenyl, 5- oder 6-gliedrige Heteroaryl oder C-Heterocyclyl bedeutet, wobei das Phenyl, 5- oder 6-gliedrige Heteroaryl oder C-Heterocyclyl gegebenenfalls bis zu 2 Substituenten, ausgewählt aus C₁₋₄-Alkyl, CF₃ und Halogen, trägt, oder, wenn Ar Phenyl bedeutet, zwei R³-Gruppen, die an benachbarte Ringatome am Ar gebunden sind, einen kondensierten 5- oder 6-gliedrigen carbocyclischen oder heterocyclischen Ring, der gegebenenfalls bis zu 3 Substituenten, ausgewählt aus Oxo, Imino und R⁵, trägt, vervollständigen können,
R⁴ H, CF₃, CH(CF₃)-Ar¹ oder Alkyl, Alkenyl, Cycloalkyl oder Cycloalkylalkyl mit bis zu 6 Kohlenstoffatomen, das gegebenenfalls substituiert ist mit Halogen, CN, CF₃, OR¹ oder NR¹R², bedeutet, oder zwei R⁴-Gruppen, die an dasselbe Stickstoffatom gebunden sind, einen heterocyclischen Ring mit bis zu 6 Gliedern, der gegebenenfalls ein zusätzliches Heteroatom, ausgewählt aus N, O und S, umfasst und der gegebenenfalls bis zu 2 Substituenten, ausgewählt aus C₁₋₄-Alkyl, CF₃, Halogen und Oxo, trägt, vervollständigen können,
R⁵ R⁴ bedeutet, das nicht H ist,
Ar¹ ein aromatisches mono- oder bicyclisches Ringsystem mit bis zu 10 Ringatomen, wovon 0-3 ausgewählt sind aus N, O und S und wobei der Rest Kohlenstoff ist, bedeutet, wobei das Ringsystem 0-3 Substituenten trägt, ausgewählt aus Halogen, CF₃ und C₁₋₄-Alkyl,
X eine Bindung, CH₂ oder CO bedeutet und
"C-Heterocyclyl" nichtaromatische heterocyclische Ringe aus 5 oder 6 Ringatomen bedeutet, wobei bis zu 2 davon ausgewählt sind aus N, O und S, wobei der Ring über ein Ring-Kohlenstoffatom gebunden ist,
wobei die neurodegenerative Erkrankung, die mit der Hyperphosphorylierung von Tau verbunden ist, ausgewählt ist aus Alzheimer-Krankheit (AD), frontotemporaler Demenz, Pick-Krankheit und Chromosom-17-gekoppeltem Parkinsonismus (FTDP-17).

2. Eine Verbindung der Formel I wie in Anspruch 1 definiert oder ein pharmazeutisch annehmbares Salz oder Hydrat davon zur Verwendung bei der Behandlung oder Prävention einer neurodegenerativen Erkrankung, die mit der Hyperphosphorylierung von Tau bei einem menschlichen Patienten verbunden ist, wobei die neurodegenerative Erkrankung, die mit der Hyperphosphorylierung von Tau verbunden ist, ausgewählt ist aus Alzheimer-Krankheit (AD), frontotemporaler Demenz, Pick-Krankheit und Chromosom-17-gekoppeltem Parkinsonismus (FTDP-17).

3. Verwendung gemäß Anspruch 1, wobei die Verbindung eine Verbindung der Formel II ist: 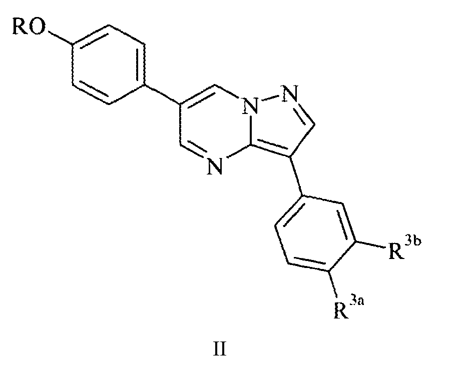 oder ein pharmazeutisch annehmbares Salz oder Hydrat davon, wobei R^{3a} und R^{3b} unabhängig H oder R³ bedeuten und R und R³ wie in Anspruch 1 definiert sind.

4. Verwendung gemäß Anspruch 1, wobei die Verbindung eine Verbindung der Formel III ist: 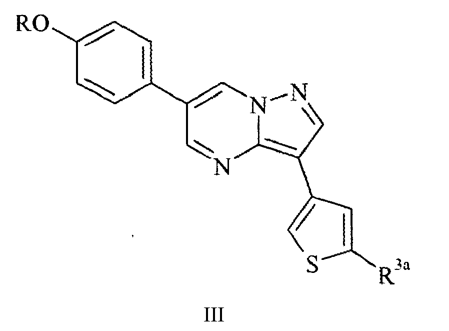 oder ein pharmazeutisch annehmbares Salz oder Hydrat davon, wobei R^{3a} H oder R³ bedeutet und R und R³ wie in Anspruch 1 definiert sind.

5. Verwendung gemäß irgendeinem der Ansprüche 1, 3 und 4, wobei R CH₂CH₂NR¹R² oder CH₂CH₂CH₂NR¹R² bedeutet, wobei R¹ und R² wie in Anspruch 1 definiert sind.

6. Eine Verbindung der Formel III: 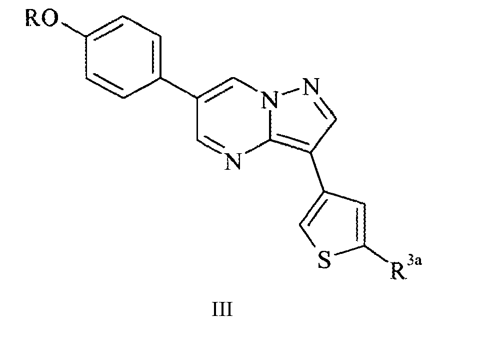 oder ein pharmazeutisch annehmbares Salz oder Hydrat davon, wobei
R C₁₋₄-Alkyl bedeutet, das mit Halogen, CN, CF₃, OR¹, NR¹R², NHPh oder NHCOC₁₋₄-Alkyl substituiert ist,
R^{3a} R³ bedeutet
und R¹, R² und R³ wie in Anspruch 1 definiert sind.

7. Eine Verbindung gemäß Anspruch 6, wobei R CH₂CH₂NR¹R² oder CH₂CH₂CH₂NR¹R² bedeutet.

8. Eine Verbindung gemäß Anspruch 6 oder Anspruch 7, wobei R^{3a} CONHR⁴ bedeutet, wobei R⁴ H oder C₁₋₄-Alkyl ist, das gegebenenfalls substituiert ist mit CF₃, OR¹ oder NR¹R².

9. Eine Verbindung gemäß Anspruch 6, wobei R^{3a} CH₂NHR⁴ oder CONHR⁴ bedeutet und R⁴ CH(CF₃)-Ar¹ bedeutet, wobei Ar¹ wie in Anspruch 1 definiert ist.

10. Eine Verbindung gemäß Anspruch 6, wobei R und R^{3a} wie in der folgenden Tabelle angegeben sind:
| **R** | **R^{3a}** |
|---|---|
| 2-(Piperidin-1-yl)ethyl | CO₂Me |
| 2-(Piperidin-1-yl)ethyl | CONHMe |
| 2-(Piperidin-1-yl)ethyl | CONHCH₂CH₂OH |
| 2-(Piperidin-1-yl)ethyl | CONH₂ |
| 2-(Piperidin-1-yl)ethyl | CONHEt |
| 2-(Piperidin-1-yl)ethyl | CONH-Isobutyl |
| 2-(Piperidin-1-yl)ethyl | CON(Me)₂ |
| 2-(Piperidin-1-yl)ethyl | CONHCH₂CH₂NH₂ |
| 2-(Piperidin-1-yl)ethyl | CO-(1-Pyrrolidinyl) |
| 2-(Piperidin-1-yl)ethyl | CO-(1-Piperidinyl) |
| 2-(Piperidin-1-yl)ethyl | CONHCH₂CF₃ |
| 2-(Piperidin-1-yl)ethyl | CONHOH |
| 2-(Piperidin-1-yl)ethyl | CONHCH₂CH₂N(Me)₂ |
| 2-(Piperidin-1-yl)ethyl | CON(Me)CH₂CH₂N(Me)₂ |
| 2-(Piperidin-1-yl)ethyl | CH₂NH-Isobutyl |
| 2-(Piperidin-1-yl)ethyl | CHO |
| 2-(Piperidin-1-yl)ethyl | CH₂NHCH₂CH₂OH |
| 2-(Piperidin-1-yl)ethyl | CH₂NHCH₂CH₂N(Me)₂ |
| 2-(Piperidin-1-yl)ethyl | CH₂N(Me)CH₂CH₂N(Me)₂ |
| 2-(Piperidin-1-yl)ethyl | 4-Isopropyl-4,5-dihydro-1,3-oxazol-2-yl |
| 2-(Dimethylamino)ethyl | CH₂CF₃ |
| 2-(Pyrrolidin-1-yl)ethyl | CH₂CF₃ |
| 2-(Piperidin-1-yl)ethyl | CH₂NHCH₂CH₂NH₂ |
| 2-(Piperidin-1-yl)ethyl | CH₂NHCH₂CF₃ |
| 2-(Piperidin-1-yl)ethyl | CH₂OH |
| 2-(Piperidin-1-yl)ethyl | 5-Methyl-4,5-dihydro-1*H*-imidazol-2-yl |

11. Eine Verbindung gemäß Anspruch 6, wobei R und R^{3a} wie in der folgenden Tabelle angegeben sind:
| **R** | **R^{3a}** |
|---|---|
| 2-(Morpholin-4-yl)ethyl | CONHCH₂CF₃ |
| 2-(4-Me-Piperazin-1-yl)ethyl | CONHCH₂CF₃ |
| 2-(Piperidin-1-yl)ethyl | CONHCH(CF₃)-(2-Furyl) |
| 2-(Piperidin-1-yl)ethyl | CONHCH(CF₃)-(2-Pyridyl) |
| 2-(Piperidin-1-yl)ethyl | CH₂NHCH(CF₃)-(2-Furyl) |
| 2-(Piperidin-1-yl)ethyl | CH₂NHCH(CF₃)-(2-Pyridyl) |
| 2-(Piperidin-1-yl)ethyl | CH(CF₃)-NH₂ |
| 2-(Piperidin-1-yl)ethyl | CONHCH(CF₃)-Me |
| 2-(Piperidin-1-yl)ethyl | CH(CF₃)-NH-Isopropyl |
| 2-(Piperidin-1-yl)ethyl | CH(CF₃)-NHCH₂-Cyclopropyl |
| 2-(Piperidin-1-yl)ethyl | CONHC(Me)₂CF₃ |
| 2-(3,3-Di-F-piperidin-1-yl)ethyl | CONHCH₂CF₃ |
| 2-(3-F-Pyrrolidin-1-yl) | CONHCH₂CF₃ |
| 2-(Piperidin-1-yl)ethyl | CONHCH(CF₃)-(3-Pyridyl) |
| 2-(3-F-Piperidin-1-yl)ethyl | CONHCH₂CF₃ |
| 2-(Piperidin-1-yl)ethyl | CONHCH(CF₃)-Isopropyl |
| 2-(3,3-Di-F-pyrrolidin-1-yl)ethyl | CONHCH₂CF₃ |
| 2-(4,4-Di-F-piperidin-1-yl)ethyl | CONHCH₂CF₃ |
| 2-(Piperidin-1-yl)ethyl | CONHCH(CF₃)-(Chinofin-5-yl) |
| 2-(Piperidin-1-yl)ethyl | CONHCH(CF₃)-(Chinofin-8-yl) |
| 2-(Piperidin-1-yl)ethyl | CH₂NHCH(CF₃)-Isopropyl |
| 2-(Piperidin-1-yl)ethyl | CONHCH(CF₃)-(1-Me-Imidazol-2-yl) |
| 2-(Piperidin-1-yl)ethyl | CONHCH(CF₃)-(4-Pyridyl) |
| 2-(Piperidin-1-yl)ethyl | CONHCH(CF₃)-(Benzthiophen-2-yl) |
| 2-AcNH-Ethyl | CONHCH(CF₃)-Isopropyl |
| Kondensiertes Tetrahydrofuran | CONHCH₂CF₃ |
| 2-Methoxyethyl | CONHCH₂CF₃ |
| 3-Methoxypropyl | CONHCH₂CF₃ |

12. Eine pharmazeutische Zusammensetzung, die eine Verbindung gemäß einem der Ansprüche 6-11 und einen pharmazeutisch annehmbaren Träger umfasst.

13. Eine Verbindung gemäß irgendeinem der Ansprüche 6-11 zur Verwendung in der Medizin.

## Revendications

1. Utilisation, dans la fabrication d'un médicament destiné au traitement ou à la prévention d'une maladie neurodégénérative associée à l'hyperphosphorylation de tau, d'un composé selon la formule I: 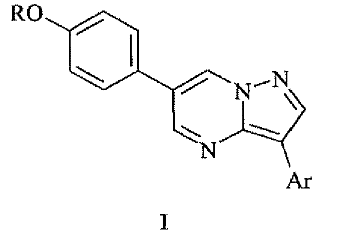 ou d'un sel ou hydrate pharmaceutiquement acceptable du même; dans lequel
R représente un alkyle C₁₋₄ qui est optionnellement substitué par un halogène, CN, CF₃, OR¹, NR¹R², NHPh ou NHCOalkyle C₁₋₄; ou bien R peut achever un cycle tétrahydrofurane fusionné;
Ar représente un phényle ou un hétéroaryle à 5 ou 6 chaînons optionnellement fusionné à un benzo, dont l'un quelconque porte optionnellement jusqu'à 3 substituants R³ sélectionnés indépendamment;
R¹ et R² représentent indépendamment H ou alkyle C₁₋₄, ou bien R¹ et R² liés au même atome d'azote peuvent achever un cycle hétérocyclique de jusqu'à 6 chaînons qui comprend optionnellement un hétéroatome supplémentaire sélectionné parmi N, O et S, lequel porte optionnellement jusqu'à 2 substituants sélectionnés parmi alkyle C₁₋₄, CN, CF₃, halogène et oxo;
R³ représente un halogène, CN, R⁵, SR⁵, X-OR⁴, X-N(R⁴)₂, CH(CF₃)-N(R⁴)₂, COR⁴, CONHOH, phényle, hétéroaryle ou C-hétérocyclyle à 5 ou 6 chaînons, lesdits phényle, hétéroaryle ou C-hétérocyclyle à 5 ou 6 chaînons portant optionnellement jusqu'à 2 substituants sélectionnés parmi alkyle C₁₋₄, CF₃ et halogène; ou bien lorsque Ar représente un phényle, deux groupes R³ attachés à des atomes cycliques adjacents sur Ar peuvent achever un cycle carbocyclique ou hétérocyclique fusionné à 5 ou 6 chaînons qui porte optionnellement jusqu'à 3 substituants sélectionnés parmi oxo, imino et R⁵;
R⁴ représente H, CF₃, CH(CF₃)-Ar¹ ou alkyle, alcényle, cycloalkyle ou cycloalkylalkyle de jusqu'à 6 atomes de carbone qui est optionnellement substitué par halogène, CN, CF₃, OR¹ ou NR¹R²; ou bien deux groupes R⁴ liés au même atome d'azote peuvent achever un cycle hétérocyclique de jusqu'à 6 chaînons qui comprend optionnellement un hétéroatome supplémentaire sélectionné parmi N, O et S et qui porte optionnellement jusqu'à 2 substituants sélectionnés parmi alkyle C₁₋₄, CF₃, halogène et oxo;
R⁵ représente R⁴ qui n'est pas H;
Ar¹ représente un système cyclique aromatique monocyclique ou bicyclique de jusqu'à 10 atomes cycliques dont 0-3 sont sélectionnés parmi N, O et S et le reste sont des atomes de carbone, ledit système cyclique portant 0-3 substituants sélectionnés parmi halogène, CF₃ et alkyle C₁₋₄;
X représente une liaison, CH₂ ou CO; et
"C-hétérocyclyle" se rapporte à des cycles hétérocycliques non aromatiques de 5 ou 6 atomes cycliques, dont jusqu'à 2 sont sélectionnés parmi N, O et S, ledit cycle étant attaché par un atome de carbone cyclique;
où ladite maladie neurodégénérative associée à l'hyperphosphorylation de tau, est sélectionnée parmi la maladie d'Alzheimer (AD), la démence frontotemporale, la maladie de Pick et le parkinsonisme lié au chromosome 17 (FTDP-17).

2. Composé de la formule I tel que défini dans la revendication 1, ou un sel ou hydrate pharmaceutiquement acceptable du même à utiliser dans le traitement ou la prévention d'une maladie neurodégénérative associée à l'hyperphosphorylation de tau chez un patient humain, où ladite maladie neurodégénérative associée à l'hyperphosphorylation de tau est sélectionnée parmi la maladie d'Alzheimer (AD), la démence frontotemporale, la maladie de Pick et le parkinsonisme lié au chromosome 17 (FTDP-17).

3. Utilisation selon la revendication 1, dans laquelle ledit composé est un composé de la formule II: 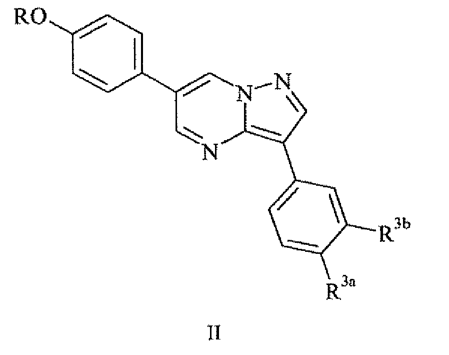 ou un sel ou hydrate pharmaceutiquement acceptable du même; où R^{3a} et R^{3b} représentent indépendamment H ou bien R³, et R et R³ sont tels que définis dans la revendication 1.

4. Utilisation selon la revendication 1, dans laquelle ledit composé est un composé de la formule III: 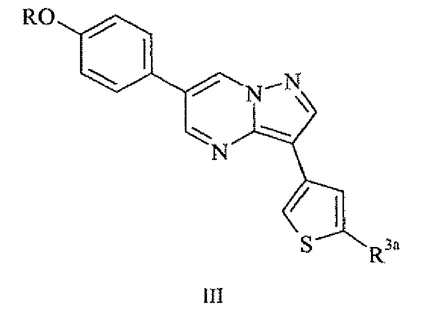 ou un sel ou hydrate pharmaceutiquement acceptable du même; où R^{3a} représente H ou bien R³, et R et R³ sont tels que définis dans la revendication 1.

5. Utilisation selon l'une quelconque des revendications 1, 3 et 4, dans laquelle R représente CH₂CH₂NR¹R² ou CH₂CH₂CH₂NR¹R², où R¹ et R² sont tels que définis dans la revendication 1.

6. Composé de la formule III: 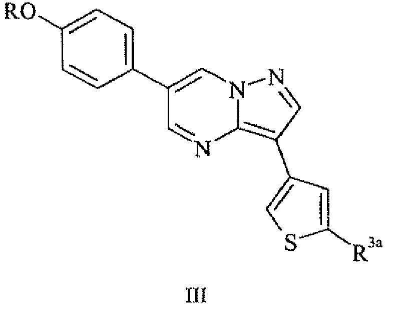 ou un sel ou hydrate pharmaceutiquement acceptable du même; dans lequel
R représente un alkyle C₁₋₄ qui est substitué par halogène, CN, CF₃, OR¹, NR¹R², NHPh ou NHCOalkyle C₁₋₄;
R^{3a} représente R³;
et R¹, R² et R³ sont tels que définis dans la revendication 1.

7. Composé selon la revendication 6, dans lequel R représente CH₂CH₂NR¹R² ou CH₂CH₂CH₂NR¹R²_{.}

8. Composé selon la revendication 6 ou la revendication 7, dans lequel R^{3a} représente CONHR⁴, où R⁴ est H ou alkyle C₁₋₄ qui est optionnellement substitué par CF₃, OR¹ ou NR¹R².

9. Composé selon la revendication 6, dans lequel R^{3a} représente CH₂NHR⁴ ou CONHR⁴ et R⁴ représente CH(CF₃)-Ar¹, où Ar¹ est tel que défini dans la revendication 1.

10. Composé selon la revendication 6, dans lequel R et R^{3a} sont tels que spécifiés dans le tableau suivant:
| R | R^{3a} |
|---|---|
| 2-(pipéridin-1-yl)éthyle | CO₂Me |
| 2-(pipéridin-1-yl)éthyle | CONHMe |
| 2-(pipéridin-1-yl)éthyle | CONHCH₂CH₂OH |
| 2-(pipéridin-1-yl)éthyle | CONH₂ |
| 2-(pipéridin-1-yl)éthyle | CONHEt |
| 2-(pipéridin-1-yl)éthyle | CONH-isobutyle |
| 2-(pipéridin-1-yl)éthyle | CON(Me)₂ |
| 2-(pipéridin-1-yl)éthyle | CONHCH₂CH₂NH₂ |
| 2-(pipéridin-1-yl)éthyle | CO-(1-pyrrolidinyle) |
| 2-(pipéridin-1-yl)éthyle | CO(1-pipéridinyle) |
| 2-(pipéridin-1-yl)éthyle | CONHCH₂CF₃ |
| 2-(pipéridin-1-yl)éthyle | CONHOH |
| 2-(pipéridin-1-yl)éthyle | CONHCH₂CH₂N(Me)₂ |
| 2-(pipéridin-1-yl)éthyle | CON(Me)CH₂CH₂N(Me)₂ |
| 2-(pipéridin-1-yl)éthyle | CH₂NH-isobutyle |
| 2-(pipéridin-1-yl)éthyle | CHO |
| 2-(pipéridin-1-yl)éthyle | CH₂NHCH₂CH₂OH |
| 2-(pipéridin-1-yl)éthyle | CH₂NHCH₂CH₂N(Me)₂ |
| 2-(pipéridin-1-yl)éthyle | CH₂N(Me)CH₂CH₂N(Me)₂ |
| 2-(pipéridin-1-yl)éthyle | 4-isopropyl-4,5-dihydro-1,3-oxazol-2-yle |
| 2-(diméthylamino)éthyle | CH₂CF₃ |
| 2-(pyrrolidin-1-yl)éthyle | CH₂CF₃ |
| 2-(pipéridin-1-yl)éthyle | CH₂NHCH₂CH₂NH₂ |
| 2-(pipéridin-1-yl)éthyle | CH₂NHCH₂CF₃ |
| 2-(pipéridin-1-yl)éthyle | CH₂OH |
| 2-(pipéridin-1-yl)éthyle | 5-méthyl-4,5-dihydro-1*H*-imidazol-2-yle |

11. Composé selon la revendication 6, dans lequel R et R^{3a} sont tels que spécifiés dans le tableau suivant:
| R | R^{3a} |
|---|---|
| 2-(morpholin-4-yl)éthyle | CONHCH₂CF₃ |
| 2-(4-Me-pipérazin-1-yl)éthyle | CONHCH₂CF₃ |
| 2-(pipéridin-1-yl)éthyle | CONHCH(CF₃)-(2-furyle) |
| 2-(pipéridin-1-yl)éthyle | CONHCH(CF₃)-(2-pyridyle) |
| 2-(pipéridin-1-yl)éthyle | CH₂NHCH(CF₃)-(2-furyle) |
| 2-(pipéridin-1-yl)éthyle | CH₂NHCH(CF₃)-(2-pyridyle) |
| 2-(pipéridin-1-yl)éthyle | CH(CF₃)-NH2 |
| 2-(pipéridin-1-yl)éthyle | CONHCH(CF₃)-Me |
| 2-(pipéridin-1-yl)éthyle | CH(CF₃)-NH-isopropyle |
| 2-(pipéridin-1-yl)éthyle | CH(CF₃)-NHCH₂cyclopropyle |
| 2-(pipéridin-1-yl)éthyle | CONHC(Me)₂CF₃ |
| 2-(3,3-di-F-pipéridin-1-yl)éthyle | CONHCH₂CF₃ |
| 2-(3-F-pyrrolidin-1-yl) | CONHCH₂CF₃ |
| 2-(pipéridin-1-yl)éthyle | CONHCH(CF₃)-(3-pyridyle) |
| 2-(3-F-pipéridin-1-yl)éthyle | CONHCH₂CF₃ |
| 2-(pipéridin-1-yl)éthyle | CONHCH(CF₃)-isopropyle |
| 2-(3,3-di-F-pyrrolidin-1-yl)éthyle | CONHCH₂CF₃ |
| 2-(4,4-di-F-pipéridin-1-yl)éthyle | CONHCH₂CF₃ |
| 2-(pipéridin-1-yl)éthyle | CONHCH(CF₃)-(quinolin-5-yle) |
| 2-(pipéridin-1-yl)éthyle | CONHCH(CF₃)-(quinolin-8-yle) |
| 2-(pipéridin-1-yl)éthyle | CH₂NHCH(CF₃)-isopropyle |
| 2-(pipéridin-1-yl)éthyle | CONHCH(CF₃)-(1-Me-imidazol-2-yle) |
| 2-(pipéridin-1-yl)éthyle | CONHCH(CF₃)-(4-pyridyle) |
| 2-(pipéridin-1-yl)éthyle | CONHCH(CF₃)-(benzothiophén-2-yle) |
| 2-AcNH-éthyle | CONHCH(CF₃)-isopropyle |
| tétrahydrofurane fusionné | CONHCH₂CF₃ |
| 2-méthoxy-éthyle | CONHCH₂CF₃ |
| 3-méthoxypropyle | CONHCH₂CF₃ |

12. Composition pharmaceutique comprenant un composé selon l'une quelconque des revendications 6-11 et un excipient pharmaceutiquement acceptable.

13. Composé selon l'une quelconque des revendications 6-11 à utiliser en médecine.
